(19) Europäisches Patentamt

European Patent Office

Office européen des brevets

(11) Publication number: 0 508 427 A2

# EUROPEAN PATENT APPLICATION

(21) Application number: 92106172.7

(22) Date of filing: 09.04.92

(51) Int. Cl.5: **G01N 33/569**, //G01N33/571, C07K15/00

(30) Priority: **09.04.91 JP 76267/91**

(43) Date of publication of application:
**14.10.92 Bulletin 92/42**

(84) Designated Contracting States:
**AT BE CH DE DK ES FR GB GR IT LI LU MC NL PT SE**

(71) Applicant: **Yanagi, Kazuo**
**5-12, Wakaba, Kukisaki-cho**
**Inashiki-gun, Ibaraki(JP)**
Applicant: **MOCHIDA PHARMACEUTICAL CO., LTD.**
**7, Yotsuya 1-chome**
**Shinjuku-ku Tokyo 160(JP)**

(72) Inventor: **Yanagi, Kazuo**
**5-12, Wakaba, Kukisaki-cho**
**Inashiki-gun, Ibaraki(JP)**
Inventor: **Inoue, Naoki**
**31-16-406, Honcho**
**Wako-shi, Saitama(JP)**
Inventor: **Harada, Shizuko**
**1-1-14, Yutaka-cho, Shinagawa-ku**
**Tokyo(JP)**

(74) Representative: **Patentanwälte Grünecker, Kinkeldey, Stockmair & Partner**
**Maximilianstrasse 58**
**W-8000 München 22(DE)**

(54) **Method and kit for measuring anti-EBNA antibodies.**

(57) This invention provides a simple immunoassay system and a measuring kit for use in the measurement of anti-EBNA antibodies, in which recombinant type EBNA proteins are used. The anti-EBNA antibody measuring kit is useful for the individual measurement of anti-EBNA-1 antibodies, anti-EBNA-2 antibodies, anti-EBNA-3 antibodies, anti-EBNA-4 antibodies, anti-EBNA-5 antibodies and anti-EBNA-6 antibodies.

Particularly, this invention provides a method for the measurement of anti-EBNA antibodies which comprises the steps of allowing anti-EBNA antibodies in a test sample to react with a recombinant type EBNA fusion protein immobilized on a support through an anti-$\beta$-galactosidase antibody, allowing the anti-EBNA antibodies to react with an enzyme-conjugated anti-EBNA antibody and measuring a signal (enzyme activity) generated from the conjugated enzyme contained in an immune complex formed by the above steps.

Also particularly, this invention provides an anti-EBNA antibody measuring kit in which an anti-$\beta$-galactosidase antibody and a recombinant type EBNA fusion protein are immobilized on a support through binding of the anti-$\beta$-galactosidase antibody to the support and binding of the recombinant type EBNA fusion protein to the anti-$\beta$-galactosidase antibody.

## Fig. 1

FIELD OF THE INVENTION

This invention relates to a method for the measurement of antibodies specific for Epstein-Barr virus (EBV) nuclear antigens (EBNA) which are formed when infected with EBV, and to a measuring kit useful for the method.

BACKGROUND OF THE INVENTION

EBV is a herpes virus which is causative of infectious mononucleosis (IM) at the time of primary infection. In certain cases, it also causes B lymphocyte-related chronic diseases. In addition, it has been indicated strongly that EBV could be the cause of nasopharyngeal carcinoma (NPC) which is a high incidence disease common for example in the southern part of China and of Burkitt's Lymphoma (BL) that is also a high incidence disease common for example in the equatorial Africa. EBV propagate itself mainly in epipharynx epithelial cells and human B lymphocytes. When a host (human) is infected, EBV causes a lifelong infection (latent infection) in which growth of the viruses and infected cells is suppressed by immunological defence mechanism and the like of the host. However, since such viruses are reactivated under certain conditions, it is considered that EBV is also related to autoimmune diseases and opportunistic lymphomatosis which occurs under immunosuppression.

When human B lymphocytes are infected with EBV, the viruses induce blastogenesis and infinite proliferation transformation (immortalization) of B lymphocytes. The immortalized cells contain EBV nuclear antigens (EBNA) which are the first-occurring antigens after EBV infection and are regarded as important factors of the immortalization of infected cells and latent viral infection.

As EBNA, at least 6 different types of polypeptides have so far been identified and named EBNA-1, EBNA-2, EBNA-3 (or EBNA-3A), EBNA-4 (or EBNA-3B), EBNA-5 (or EBNA-LP or EBNA-4) and EBNA-6 (or EBNA-3C) (designation of these polypeptides differs among main research groups).

EBNA-1 has a molecular weight of 65 to 75 kDa and takes an essential role in the replication of EBV-DNA in immortalized B cells. EBNA-2 has a molecular weight of 82 kDa and is essential for the immortalization of B cells. Functions of other EBNA polypeptides are not clear yet. When the function of EBNA-1 and EBNA-2 to control growth of viruses and host cells is taken into consideration, it seems probable that each of EBNA-3 to 6 has each own inherent function and takes important roles in the lymphocyte abnormality specific for EBV-related diseases and infinite proliferation transformation of infected cells.

In consequence, it is expected that development of a method which can measure antibody titers against each EBNA polypeptide individually and accurately will render possible more detailed analysis of diseases under suspicion of EBV-involvement. In addition, such a measuring method will find versatile use in such applications as definite diagnosis of a disease, understanding of the condition of a disease and prevention of a disease.

An anti-complement immunofluorescence technique (ACIF) is generally used for the measurement of anti-EBNA antibody titers in blood, in which EBNA-producing EBV-infected human B cells are used. However, since all types of the EBNA molecules are produced in such a state of the cells, antibody titers against each of the 6 EBNA polypeptides cannot be measured individually by this method. In addition, this measuring method requires complex handling and great skill. What is more, it is known that judgement of the measured results by this method is not always clear and sometimes swayed by an operator's own feeling.

Recently, transformed cell lines which can express EBNA-1 and EBNA-2 have been established by means of recombinant DNA techniques and gene transduction methods, and immune responses of host cells to each EBNA polypeptide have been analyzed by an immunofluorescence analysis (IFA) and ACIF making use of these cell lines (Henle W. *et al.*, Proc. Natl. Acad. Sci., USA, 84, p.570, 1987; Seigneurin J.M. *et al*, Int. J. Cancer, 40, p.349, 1987; Miller G. *et al.*, J. Infec. Dis.,156, p.26, 1987).

For example, Henle *et al.* have measured antibody titers against antigens in sera of patients of acute stage IM and chronic active EB virus infection, by means of ACIF using a cell line obtained as a source of EBNA-1 antigens by incorporating a *Bam*HI-K fragment of EBV(B95-8)-DNA into mouse 3T3 fibroblasts and another cell line obtained as a source of EBNA-2 antigens by incorporating a *Bam*HI-WYH fragment into human DG75 cells. By comparing antibody titers against antigens of the two diseases, they have found that patterns of the appearance, increment and disappearance of the antibody titers differed depending on the type of diseases.

However, measurement of anti-EBNA antibody titers by ACIF or IFA making use of such animal cells requires complex handling, as well as skilled techniques because the results are judged by microscopic observation. In addition, simultaneous handling of a large number of samples cannot be made easily, and an automatic inspection system is not available yet. Moreover, the prior art measuring method in which EBV-infected latent period human B cells are used has a serious disadvantage in that, when serum samples of autoimmune disease patients are measured, proper judgement of the results cannot be made in some cases due to strong interference by autoantibodies specific for cellular components.

In view of the above, it becomes an object of the present invention to provide a simple immunoassay system and a measuring kit for use in the measurement of anti-EBNA antibodies, in which recombinant type EBNA protein is used. Another object of the present invention is to provide an anti-EBNA antibody measuring kit which is useful for the individual measurement of anti-EBNA-1 antibodies, anti-EBNA-2 antibodies, anti-EBNA-3 antibodies, anti-EBNA-4 antibodies, anti-EBNA-5 antibodies and anti-EBNA-6 antibodies.

## SUMMARY OF THE INVENTION

Taking the aforementioned prior art problems into consideration, the inventors of the present invention have conducted intensive studies and, as the results, accomplished the present invention by developing a simple and practical method for the measurement of anti-EBNA antibodies in test samples, as well as a measuring kit for use in the measurement, making use of a recombinant type fusion protein containing any one of EBNA-1 to 6 which are now obtainable in a large quantity by recombinant DNA techniques or using recombinant type EBNA-1 to 6.

According to a first aspect of the present invention, there is provided a method for measuring anti-EBNA antibodies which comprises using a recombinant type EBNA-based protein.

According to a second aspect of the present invention, there is provided an anti-EBNA antibody measuring kit for use in the operation of the measuring method of the first aspect of the present invention, which comprises a recombinant type EBNA-based protein.

According to a third aspect of the present invention, there is provided a panel type anti-EBNA antibody measuring method which can measure at least two different types of anti-EBNA antibodies in one test sample simultaneously and individually and kit which is useful for the measuring method.

## BRIEF DESCRIPTION OF THE INVENTION

Fig. 1 is a view showing positions of *Bam*HI fragment and each subfragment on EBNA-DNA.
Fig. 2 is a pictorial view showing immune complexes.
Fig. 3 is a pictorial view showing immune complexes.
Fig. 4 is a pictorial view showing immune complexes.
Fig. 5 is a pictorial view showing immune complexes.
Fig. 6 is a pictorial view showing immune complexes.
Fig. 7 is a pictorial view showing immune complexes.
Fig. 8 is a pictorial view showing immune complexes.
Fig. 9 is a pictorial view showing immune complexes.
Fig. 10 is a pictorial view showing immune complexes.
Fig. 11 is a view showing construction steps of plasmid pRIT-Y1.
Fig. 12 is a view showing a result of immunoblotting of the reactivity of EBNA fusion protein samples with EBV-positive human serum.
Fig. 13 is a graphical representation showing a result of the measurement of anti-EBNA-1 antibodies in a serum sample collected from a chronic rheumatoid arthritis (RA) patient and two serum samples collected from healthy volunteers.
Fig. 14 is a correlation diagram showing results of the measurement of anti-EBNA-1 antibody titers in 36 serum samples collected from healthy volunteers, by a prior art method and the method of this invention.
Fig. 15 is a graphical representation showing distributions of IgG antibody titers in a serum sample of healthy volunteer against K2 (EBNA-1) fusion protein, H7 (EBNA-2) fusion protein, S8 (EBNA-3) fusion protein and N4 (EBNA-4) fusion protein.
Fig. 16 is a graphical representation showing a distribution of IgG antibody titers in a serum sample of healthy volunteer against protein Y1 (EBNA-2).
Fig. 17 is a graphical representation showing a result of the measurement of anti-EBNA-1 antibodies in an anti-VCA antibody positive or negative serum sample (200 times dilution) collected from a healthy volunteer, measured by means of a sandwich type ELISA using K2 (EBNA-1) fusion protein.

Fig. 18 is a correlation diagram showing results of the measurement of anti-EBNA-1 antibody titers in a single sample, by means of an indirect type ELISA using SII (EBNA-1) fusion protein and by a sandwich type ELISA using K2 (EBNA-1) fusion protein.

DETAILED DESCRIPTION OF THE INVENTION

The following describes construction of the present invention in detail.

Firstly, technical terms as used herein are explained.

The term "measurement" as used herein means qualitative or quantitative detection of the presence of anti-EBNA antibodies in test samples.

The each of the terms "recombinant type EBNA-based protein", "recombinant type EBNA-based protein x" and "recombinant type EBNA-based protein y" as used herein means protein which contains at least a portion of EBNA produced by means of recombinant DNA techniques. As will be described later, two types of recombinant type EBNA-based protein are used in one measuring method or one measuring kit in some cases. In such cases, the two types are defined as x and y. In general, "recombinant type EBNA-based protein x" and "recombinant type EBNA-based protein y" may be one and the same, but may be different from each other provided that they have the same antigen epitope.

The term "at least a portion of EBNA" as used herein as a composing element of "recombinant type EBNA-based protein" means at least a portion of the protein structure of any one of EBNA-1 to EBNA-6.

In consequence, the "recombinant type EBNA-based protein" may be a protein which contains the complete structure of at least one of EBNA-1 to EBNA-6, a fusion protein that comprises at least a portion of EBNA and other protein than EBNA, or an EBNA analog. Also to be included in the "recombinant type EBNA-based protein" are a protein which contains protein structures of at least two different EBNA types and a protein that contains at least two structural portions of one EBNA type.

According to the present invention, it is desirable to use a recombinant type EBNA-based protein which contains at least a portion of one EBNA type, a portion effective as its antigen binding site, and which does not contain non-specific regions. Such a type of protein is useful as an antigen source for the production of low cross-reactive antibodies specific for one type of EBNA.

The term "fusion protein" as used herein to be included in the recombinant type EBNA-based protein means a fusion protein which contains at least a portion of the aforementioned EBNA and a so-called "carrier protein".

A material to be used in the present invention as the carrier protein moiety of the fusion protein may be selected from various sources, provided that they are expressed stably in host cells, but preferably from those materials which have certain activities useful as markers when the fusion protein is recovered and/or which can yield respective antibodies.

Illustrative examples of the carrier protein include $\beta$-galactosidase, protein A, $\beta$-lactamase, human growth factor, alkaline phosphatase, λN protein, ompA, ompC, chloramphenicol acetyl transferase (CAT) and the like, of which $\beta$-galactosidase and protein A are particularly preferred.

The term "EBNA analog" as used herein to be included in the recombinant type EBNA-based protein means a substance which contains at least one of regions that can be used as antigen binding sites of EBNA.

The recombinant type EBNA-based protein may be produced by any process making use of recombinant DNA techniques. The following describes a preferred example of the production process.

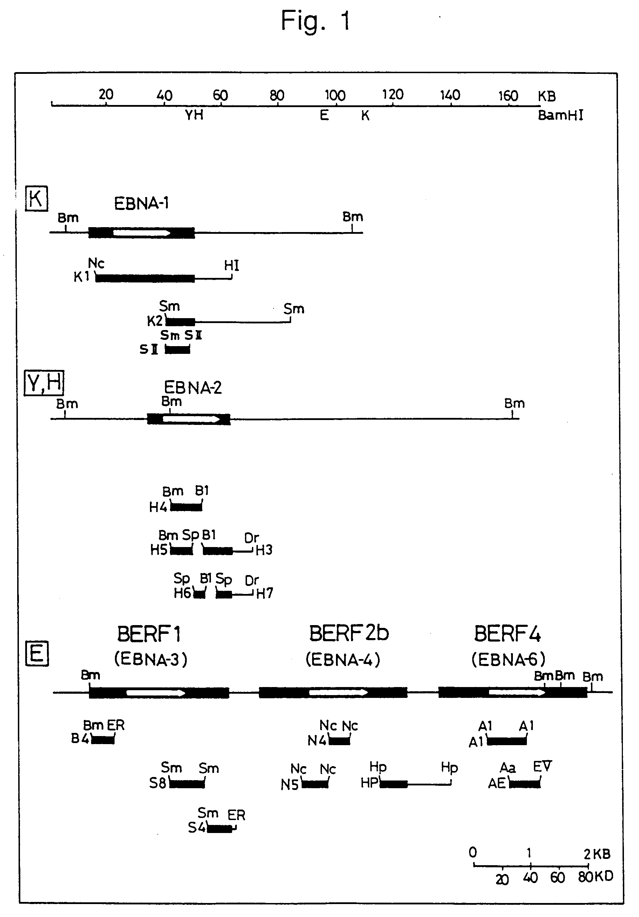

Firstly the type of EBNA and its portion to be used as "at least a portion of EBNA" are decided. In this instance, DNA sequences of an EBV-DNA *Bam*HI fragment and its subfragments isolated by digesting the fragment with various restriction enzymes, which have been reported by Baer *et al.* (Nature,.310, pp.207 - 211, 1984), as shown in Table A and Fig. 1 may serve as a good reference.

Table A

| Types of EBNA | *Bam*HI fragments | Corresponding translation regions | DNA regions to be selected | Subfragments |
|---|---|---|---|---|
| EBNA-1 | K | BKRF1 | 109300 - 111539 | K2 |
| EBNA-1 | K | BKRF1 | 109300 - 109806 | SII |
| EBNA-2 | Y,H | BYRF1 | 48849 - 49475 | H4 |
| EBNA-2 | Y,H | BYRF1 | 49649 - 50306 | H7 |
| EBNA-2 | Y,H | BYRF1 | 48504 - 48848 | Y1 |
| EBNA-3 | E | BERF1 | 94029 - 94784 | S8 |
| EBNA-4 | E | BERF2b | 96859 - 97216 | N4 |
| EBNA-6 | E | BERF4 | 99612 - 100357 | A1 |

In the above table, the term "DNA regions to be selected" is expressed by DNA positions of EBV in accordance with Baer *et al*. (already mentioned in the foregoing). The term "*Bam*HI fragments" means names of fragments each of which encoding respective type of EBNA when the EBV(B95-8) DNA fragment is digested with *Bam*HI. Also, the term "Corresponding translation regions" means, in the case of BKRF1 for example, the first region of right-forward open reading frame on the *Bam*HI-K fragment.

In Fig. 1, subfragments each of which consisting solely of a portion of EBNA are shown, as well as other subfragments containing portions that do not encode EBNA in the EBV-DNA. Also, in Fig. 1, abbreviations shown on both sides of each subfragment indicate names of the following restriction enzymes which are used to obtain the subfragment.

Aa, *Aat*II; Al, *Alu*I; Bl, *Bal*I; Bm, *Bam*HI; Dr, *Dra*I; ER, *Eco*RI; EV, *Eco*RV; Hi, *Hind*III; Hp, *Hpa*I, Nc, *Nco*I; Pv, *Pvu*II; SII, *Sac*II; Sm, *Sma*I; Sp, *Sph*I

Next, a DNA fragment containing the thus selected region (a DNA fragment which encodes at least a portion of EBNA) is prepared.

A DNA fragment which encodes at least a portion of EBNA may be prepared by any available means for example by synthesizing it chemically or by isolating it from the EBV-DNA, particularly from DNA fragments in EBV-positive cells. Examples of such EBV-positive cells include those of cell lines B95-8, P3HR1, Jijoye, Raji and the like, of which cells of strain B95-8 are particularly preferred.

DNA samples may be digested with restriction enzymes, when an EBV-DNA fragment containing a DNA fragment coding for any one of the EBNA-1 to EBNA-6 is isolated from the EBV-DNA, or when subfragments such as those shown in Table A are isolated from the EBV-DNA fragment.

Chemical synthesis of a DNA fragment which encodes at least a portion of EBNA may be carried out in the usual way, for example by selecting a sequence of a region of interest from the EBNA sequence, dividing the selected sequence into proper length of fragments when required and then chemically synthesizing corresponding oligomers using an automatic DNA synthesizer such as model 381A manufactured by Applied Biosystems, Inc.

When a DNA fragment coding for at least a portion of EBNA is obtained by either of these means, the fragment is inserted into an appropriate cloning vector in combination with proper linkers DNA sequences as restriction enzyme recognition sites and the like. For example, firstly, necessary sequences are selected from a DNA sequence coding for amino acid sequence of a carrier protein to be used, DNA sequences of appropriate vector and linkers, a promoter sequence, a repressor sequence, an SD sequence, a terminator sequence, a termination codon, a translation initiation codon, restriction enzyme recognition sites for cloning use and the like. Thereafter, oligonucleotides corresponding to the thus selected sequences are prepared by chemical synthesis or the like means, the synthesized oligonucleotides are ligated with a DNA fragment which encodes at least a portion of EBNA and then the thus ligated product is inserted into a proper site of a cloning vector such as a plasmid vector, a phage vector, a virus vector or the like.

Illustrative examples of plasmid vectors include pBR322, pUC18, pUC19 and the like, those of phage vectors include λgt10, λgt11 and the like and those of virus vectors include pMAM-neo, pKCR and the like. Of these cloning vectors, preferred examples are lambda phage vectors and plasmid vectors which hardly cause DNA defections and are used widely as materials in recombinant DNA experiments. Particularly preferred is a λgt11 or the like lambda phage vector which contains β-galactosidase structural gene (*lac*Z) in which a DNA fragment encoding at least a portion of EBNA is inserted. Also particularly preferred is a plasmid vector pRIT2T (Nilsson, B. *et al*., EMBO J., 4, pp.1075 - 1080, 1985) which contains a region coding for protein A and in which a DNA fragment encoding at least a portion of EBNA is inserted.

A DNA fragment which encodes at least a portion of EBNA or a variation of the DNA fragment to which other DNA sequences are attached may be introduced into a cloning vector by usually used means, for example in accordance with the techniques disclosed in *Molecular Cloning*, a *laboratory manual* (T. Maniatis *et al.*, Cold Spring Harbor Laboratory, 1982 or 1990).

In this way, a vector for use in the transformation of a host is obtained. Preferably, such a vector (recombinant DNA) may have additional DNA sequences which encode a promoter sequence, a repressor sequence, an SD sequence (or an optional ribosome binding site) and the like which are necessary for the expression of EBNA-based protein in a host transformed with the vector. Especially, when a virus vector is used as the cloning vector, it may contain an RNA splicing region and a poly(A) addition signal. In this instance, the DNA sequences coding for a promoter sequence, a repressor sequence, an SD sequence and the like are not limited, provided that they can exhibit their functions in a host to be used.

Also preferably, such a vector (recombinant DNA) may contain not only a DNA sequence which encodes a carrier protein but also a sequence that is necessary for the effective expression of the EBNA-based protein, thereby rendering possible improvement of the productivity of the EBNA itself.

When a proper vector (recombinant DNA) is obtained, an appropriate host is transformed with the vector to isolate a transformant. In this instance, the term "transformation" as used herein should be understood in a broad sense. In other words, the term "transformation" as used herein means that a character of a host is altered by a vector and the host acquires a new property as the result. In consequence, transduction effected by lysogenization of a host with a phage vector and other techniques is also included in the term "transformation" as used herein in a broad sense.

A transformant may be obtained by introducing a proper vector (recombinant DNA) into an appropriate host cell making use of generally known techniques such as calcium chloride technique, a method in which a calcium phosphate-DNA complex is used, microinjection, electroporation and the like.

Host cells to be introduced with the vector may be selected from either eukaryotic cells such as COS cells, CHO cells, yeast and the like or prokaryotic cells such as *E. coli*, *B. subtilis* and the like, provided that they are suitable for use in the expression of EBNA-based protein, of which *E. coli* are particularly preferred.

When the vector does not contain a base sequence necessary for the expression of EBNA-based protein, it is preferable to use a host which is possessed of a gene containing such a base sequence.

It is effective to use a host and a vector in such a combination that they can exhibit their functions mutually. Preferred examples of the combination of host cells with cloning vectors include a combination of COS-7 cells with an expression vector containing the simian virus 40 (SV40) early promoter and a combination of *E. coli* HB101 cells with an expression vector containing a DNA sequence which encodes a tryptophan promoter of plasmid pBR322 origin and a tryptophan SD sequence.

The inventors of the present invention have isolated *E. coli* strains transformed with vectors containing DNA fragments coding for the EBNA-based protein to be used in the present invention. These transformants have been deposited by the present inventors on December 13, 1990, in Fermentation Research Institute, Agency of Industrial Science and Technology 1-3, Higashi 1 chome, Tsukuba-shi, Ibaraki-ken 305, Japan, and have been assigned the designations as FERM P-11902 (expresses EBNA-6), FERM P-11903 (expresses EBNA-2), FERM P-11904 (expresses EBNA-1), FERM P-11905 (expresses EBNA-4), FERM P-11906 (expresses EBNA-3) and FERM P-11907 (expresses EBNA-2).

A transformant thus isolated is cultured to allow it to produce fusion protein.

Culturing of a transformant, namely host cells transformed with recombinant DNA, may be effected by generally used means for the culturing of microorganisms or animal cells, in accordance with the procedure disclosed for instance in *Seibutsu Kagaku Kogaku* (or Biochemical Engineering; S. Aiba, 1976, Tokyo University Press) or in *Virus Jikkengaku* - A Review (or Viral Experiments; second edition by Gakuyu-kai, National Institute of Health, 1976, Maruzen).

The thus produced EBNA-based protein by the transformed host cells is recovered by purifying it from the cultured mixture. More illustratively, it is desirable to isolate the EBNA-based protein from the cultured cells when the protein is produced intracellularly or from the cultured filtrate when produced extracellularly.

Recovery of the EBNA-based protein from the cultured mixture may be carried out in the light of various generally used protein purification means which have been disclosed in many reports and books such as *Seikagaku Jikken Koza* (or Biochemical Experiments; vol. 1, Protein Chemistry, 1976, edited by The Japanese Biochemical Society, Tokyo Kagaku Dojin).

Purification of the EBNA-based protein from the cultured mixture may be carried out by selecting appropriate means from for instance salting-out, ultrafiltration, isoelectric precipitation, gel filtration, ion exchange chromatography, hydrophobic chromatography, affinity chromatography, chromatofocusing, adsorption chromatography, reverse phase chromatography, high performance liquid chromatography and the like.

When the EBNA-based protein is in the form of a fusion protein, its carrier protein moiety may if necessary be separated from the EBNA-based protein by means of enzymatic or chemical treatment.

As an agent for use in the enzymatic treatment, a proper enzyme may be selected from peptidases such as collagenase, thrombin, blood coagulation factor Xa, *Achromobacter* protease I (API) and the like, depending on a site to be digested. For example, a C-terminal side moiety of a lysine residue can be cut out from a fusion protein when API is used, and a C-terminal side moiety of an arginine residue when thrombin is used.

BrCN may be used most effectively as an agent for use in the chemical treatment. By the use of BrCN, a C-terminal side moiety of a methionine residue can be cut out.

In either case of the enzymatic and chemical treatments, it is necessary to select a treating agent in such a way that the site to be cut by the agent is not contained in the protein of interest, that is, EBNA.

For the purpose of improving efficiency of the separation of a carrier protein moiety of a fusion protein from EBNA by any of the above treating means, it is desirable to construct a vector which contains a DNA fragment coding for a carrier protein and at least a portion of EBNA in such a manner that a specific base sequence is inserted between a DNA sequence coding for the carrier protein and another DNA sequence encoding at least a portion of EBNA. When a transformant is obtained by transforming a host with a vector constructed in this manner, an EBNA-carrier fusion protein produced by the transformant can accurately be cut into the carrier protein and EBNA by the action of an enzyme or a chemical substance which recognizes and cuts the inserted specific base sequence.

Thus, a single EBNA (partial or complete) or EBNA-carrier fusion protein can be obtained as the EBNA-based protein by recombinant DNA techniques. In addition, since a carrier protein moiety in a EBNA-carrier fusion protein can be separated from EBNA, single EBNA can be obtained even when a transformant produces the fusion protein.

As will be discussed later, a single EBNA protein and an EBNA-carrier fusion protein may be used as the EBNA-based protein selectively depending on the object and use.

The term "antibody a" as used herein means anti-EBNA antibodies and/or anti-carrier protein antibodies.

The "antibody a" may be in the form of polyclonal or monoclonal antibodies which can be obtained using an appropriate immunogen in accordance with known procedures disclosed for instance in *Men-eki Jikken Sosa-ho* (or Immunological Experiments and Procedures; Vol. I to IX, 1980, The Japanese Society of Immunology).

Examples of the immunogen for use in the preparation of "antibody a" include any one of EBNA-1 to EBNA-6, a fusion protein containing any one of EBNA-1 to EBNA-6 and a carrier protein ($\beta$-galactosidase, protein A or the like).

The source of "antibody a" is not particularly limited, and can be obtained from a goat, a mouse, a rabbit, a rat, a guinea pig and the like. In addition, when the "antibody a" is an anti-carrier protein antibody, commercial articles are available.

The term "antibody b" means anti-antibodies which can react with anti-EBNA antibodies in a test sample or with the aforementioned "antibody a".

Like the case of the "antibody a", the "antibody b" may be in the form of polyclonal or monoclonal antibodies which can be obtained using an appropriate immunogen in accordance with known procedures disclosed for instance in *Men-eki Jikken Sosa-ho* (or Immunological Experiments and Procedures; Vol. I to IX, 1980, The Japanese Society of Immunology).

The "antibody b" to be used in the present invention may be selected from optional antibodies, provided that they can satisfy the aforementioned conditions, but may preferably be anti-human immunoglobulin antibodies, more preferably anti-human immunoglobulin Fc-specific antibodies.

When a specific class or subclass of anti-EBNA antibodies in a test sample are measured, anti-immunoglobulin antibodies which correspond to the class or subclass to be measured may be used as the "antibody b". For example, when only IgA among anti-EBNA antibodies is measured, anti-IgA antibodies may be used as the "antibody b".

The source of "antibody b" is not particularly limited, and can be obtained from a goat, a mouse, a rabbit, a guinea pig and the like. In addition, it may be understood easily that, though not an antibody, protein A which has a function to undergo specific reaction with the Fc moiety of immunoglobulins can be used in the present invention as an agent having the same function of "antibody b".

The term "antibody c" as used herein means antibodies which can bind to EBNA in competition with anti-EBNA antibodies in a test sample.

Like the case of the "antibody a" and "antibody b", the "antibody c" may be in the form of polyclonal or monoclonal antibodies, but preferably in the polyclonal form because the "antibody c" should be competitive with anti-EBNA antibodies in a test sample. In any case, it can be obtained using an appropriate immunogen in accordance with known procedures disclosed for instance in *Men-eki Jikken Sosa-ho* (or Immunological Experiments and Procedures; Vol. I to IX, 1980, The Japanese Society of Immunology).

Among the materials described above, "recombinant type EBNA-based protein y", "antibody a", "antibody b" and "antibody c" are proteins to which labeling compounds can be bound, because these proteins are used in some cases by labeling them with appropriate labeling compounds which are described in the following.

The term "labeling compound" as used herein means a compound which generates certain measurable signals directly or indirectly.

Examples of labeling compounds which directly generate measurable signals include radioactive isotopes, fluorescent materials, chemiluminescent materials and the like, and those which indirectly generate measurable signals include enzymes (coloring of substrates for example) and the like.

Illustrative examples of radioactive isotopes include $^{125}$I, $^{14}$C and the like, those of fluorescent materials include fluorescene, umbelliferone, rare earth metal chelates and the like, those of chemiluminescent materials include luminol, isoluminol, acridinium esters, dioxetanes and the like, and those of enzymes include horseradish peroxidase, alkaline phosphatase and the like.

The "labeling compound" to be used in the present invention is selected to improve measuring sensitivity of anti-EBNA antibodies in test samples, but preferably from non-radioactive compounds including enzymes, fluorescent materials and chemiluminescent materials in view of easy measuring operation and the like.

Binding of the "labeling compound" with "antibody a", "antibody b", "antibody c" or "recombinant type EBNA-based protein y" may be effected in accordance with known procedures disclosed for instance in *Kohso Men-eki Sokutei-ho* (or Enzyme Immunoassay; E. Ishikawa *et al.*, 1987, third edition, Igaku Shoin) or by optionally modifying these procedures.

As to the "labeled antibody b", corresponding commercial articles may be used.

The term "support" as used herein means a material to which "antibody a", "antibody b" or "EBNA-based protein" is bound physically or chemically to form a solid phase.

Illustrative examples of the "support" include microplates, beads, latexes, tubes, red blood cells, magnetic microparticules, films and the like, of which microplates are particularly preferred.

The term "anti-EBNA antibody" as used herein means an antibody specific for a partial or complete molecules of at least one of EBNA-1, EBNA-2, EBNA-3, EBNA-4, EBNA-5 and EBNA-6.

The term "test sample" as used herein means a liquid which has a possibility of containing at least one of the anti-EBNA-1 antibody, anti-EBNA-2 antibody, anti-EBNA-3 antibody, anti-EBNA-4 antibody, anti-EBNA-5 antibody and anti-EBNA-6 antibody.

Illustrative examples of the "test sample" include body fluids such as serum, plasma, whole blood, urine and the like and extracted solution from biological materials. The "test sample" mainly used in the practice of the present invention include blood samples, especially serum or plasma samples.

Thus, "technical terms" in relation to the present invention have been described. Next, a measuring method of anti-EBNA antibodies of a first aspect of the present invention and a measuring kit for use in the measurement of anti-EBNA antibodies of a second aspect of the present invention are described in the following with reference to the figures as per attached hereto.

The first aspect of the present invention is a method for the measurement of anti-EBNA antibodies which comprises using a recombinant type EBNA-based protein, and the second aspect of the present invention is a measuring kit for use in the measurement of anti-EBNA antibodies which is used for the practice of the measuring method of the first aspect of the present invention.

Though the first and second aspects of the present invention may include various variations, their typical examples are described in the following with reference to the figures as per attached hereto.

Firstly, examples in which the measurement is based on the sandwich method are described.

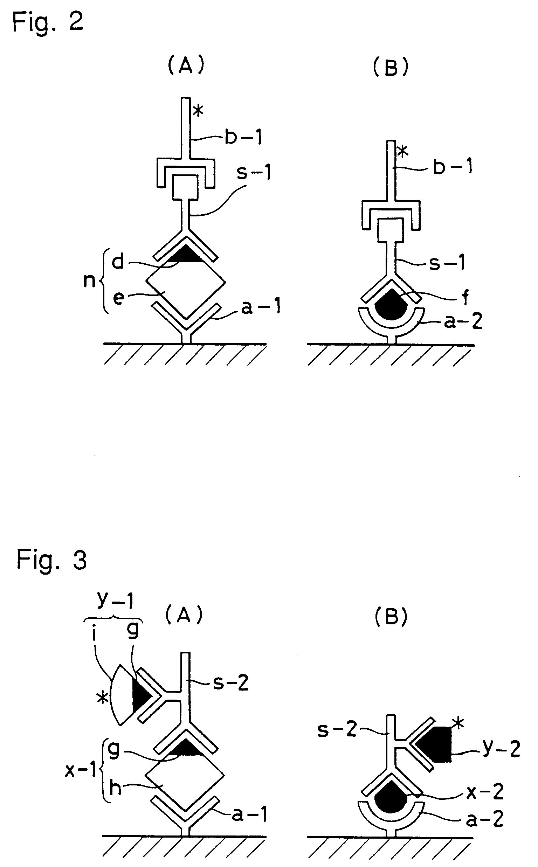

Fig. 2(A) is a pictorial view showing an immune complex comprising a solid phase antibody a-1, a recombinant type EBNA fusion protein n, an anti-EBNA antibody s-1 and a labeled antibody b-1 in that order, which is formed through the steps of:

allowing the recombinant type EBNA fusion protein n (including an EBNA protein d and a carrier protein e) made into a solid phase on a support through the antibody a-1 as an anti-carrier protein antibody to react with the anti-EBNA antibody s-1 in a test sample, and

allowing said anti-EBNA antibody s-1 to react with the antibody b-1 labeled with a labeling compound; or through the steps of:

allowing said antibody a-1 immobilized on the support to react with said EBNA fusion protein n,

allowing said EBNA fusion protein n n to react with the anti-EBNA antibody s-1 in a test sample, and

9

allowing said anti-EBNA antibody s-1 to react with the antibody b-1 labeled with a labeling compound.

Fig. 2(B) is a pictorial view showing an immune complex comprising a solid phase antibody a-2, a recombinant type EBNA protein (or an analog thereof) f, an anti-EBNA antibody s-1 and a labeled antibody b-1 in that order, which is formed through the steps of:

allowing the recombinant type EBNA protein (or an analog thereof) f immobilized on a support through the antibody a-2 as an anti-EBNA antibody to react with the anti-EBNA antibody s-1 in a test sample, and

allowing said anti-EBNA antibody s-1 to react with the antibody b-1 labeled with a labeling compound; or through the steps of:

allowing said antibody a-2 made into a solid phase on the support to react with said EBNA protein (or an analog thereof) f,

allowing said EBNA protein f to react with the anti-EBNA antibody s-1 in a test sample, and

allowing said anti-EBNA antibody s-1 to react with the antibody b-1 labeled with a labeling compound.

A measuring method (part 1) of the first aspect of the present invention in which the immune complex shown in Fig. 2(A) is formed comprises the steps of:

forming the immune complex which comprises a solid phase antibody a-1, a recombinant type EBNA fusion protein n, an anti-EBNA antibody s-1 and a labeled antibody b-1 in that order, prepared through the steps of allowing the recombinant type EBNA fusion protein n (including an EBNA protein d and a carrier protein e) made into a solid phase on a support through the antibody a-1 as an anti-carrier protein antibody to react with the anti-EBNA antibody s-1 in a test sample, and then allowing said anti-EBNA antibody s-1 to react with the antibody b-1 labeled with a labeling compound; and

measuring a signal generated from a labeling compound contained in said immune complex.

More particularly, the measuring method (part 1) is effected for example by mixing a test sample with a support to which said fusion protein n is made into a solid phase through said antibody a-1, binding said fusion protein n with the anti-EBNA antibody s-1 in the test sample, adding said labeled antibody b-1 to the reaction system to bind the labeled antibody b-1 to said anti-EBNA antibody s-1 and then measuring a signal generated from a labeling compound contained in the immune complex.

A measuring method (part 2) of the first aspect of the present invention in which the immune complex shown in Fig. 2(A) is formed comprises the steps of:

forming the immune complex which comprises a solid phase antibody a-1, a recombinant type EBNA fusion protein n, an anti-EBNA antibody s-1 and a labeled antibody b-1 in that order, prepared through the steps of allowing said antibody a-1 made into a solid phase on a support to react with said EBNA fusion protein n, allowing said EBNA fusion protein n to react with anti-EBNA antibody s-1 in a test sample, and allowing said anti-EBNA antibody s-1 to react with the antibody b-1 labeled with a labeling compound; and

measuring a signal generated from a labeling compound contained in said immune complex.

More particularly, the measuring method (part 2) is effected for example by adding said fusion protein n to a support to which said antibody a-1 is bound as a solid phase, binding said fusion protein n with said antibody a-1, adding a test sample to the reaction system, binding said fusion protein n with the anti-EBNA antibody s-1 in the test sample, adding said labeled antibody b-1 to the reaction system to bind the labeled antibody b-1 to said anti-EBNA antibody s-1 and then measuring a signal generated from a labeling compound contained in the immune complex.

Each of the measuring methods (part 1) and (part 2) includes variations in which the binding order is changed or at least two of the binding reactions are carried out at the same time.

For example, in the case of the measuring method (part 2), it may be effected by firstly binding the anti-EBNA antibody s-1 in a test sample to the labeled antibody b-1, followed by further binding of EBNA fusion protein n to form an immune complex which comprises the recombinant type EBNA fusion protein n, the anti-EBNA antibody s-1 and the labeled antibody b-1 in that order, subsequently binding the thus formed immune complex to the solid phase antibody a-1 on a support, and finally measuring a signal generated from a labeling compound contained in the immune complex. It may be effected also by firstly subjecting all of the anti-EBNA antibody s-1 in a test sample, the labeled antibody b-1, the EBNA fusion protein n and the solid phase antibody a-1 to simultaneous binding reaction, thereby forming an immune complex which comprises the solid phase antibody a-1, the recombinant type EBNA fusion protein n, the anti-EBNA antibody s-1 and the labeled antibody b-1 in that order, and then measuring a signal generated from a labeling compound contained in the immune complex. These variations are included in the measuring method (part 2) of the first aspect of the present invention.

Measuring methods (part 3) and (part 4) of the first aspect of the present invention in which the immune complex shown in Fig. 2(B) is formed may be understood easily when the antibody a-1 and the EBNA fusion protein n described above in relation to the immune complex of Fig. 2(A) are replaced by an antibody a-2 and an EBNA protein (or an analog thereof) f, respectively.

In the measuring methods (part 1 to part 4) described above, it is desirable to carry out each binding reaction under proper conditions, generally at a temperature of approximately from 4 to 40°C for a period of approximately from 30 minutes to 24 hours, preferably at a temperature of approximately from 20 to 37°C for a period of approximately from 30 minutes to 2 hours.

If necessary, a washing step making use of a buffer solution and the like may be added optionally to the measuring methods (part 1 to part 4).

When used in such immune reaction systems, it is desirable to select a buffer solution which can prevent the immune reaction from interference by substances other than anti-EBNA antibodies, such as serum components, because such an interference occurs frequently when serum and the like are used as test samples, or to use a buffer solution containing an component which can inhibit interference of the immune reaction. Examples of such components which can inhibit interference of the immune reaction include animal sera, salts and the like, more particularly, gelatin, albumin, skim milk, detergents and the like.

When a test sample which contains immunoglobulin in addition to anti-EBNA antibodies is used, such as serum, plasma, pleural effusion, ascites or the like, and anti-human immunoglobulin antibodies are used as the labeled antibody b in a step next to the adding step of a test sample-containing solution, it is particularly desirable to insert a washing step between these two steps. Such a washing step is employed to prevent inhibition of the binding reaction of anti-human immunoglobulin antibodies with anti-EBNA antibodies caused by the presence of a large quantity of immunoglobulin molecules in the test sample.

A method for the measurement of signals may be selected optionally depending on the type of labeling compounds. For example, when the labeling compound is an enzyme, its enzymatic activity is measured using a substrate, a chromogen and the like. When the labeling compound is a fluorescent or a chemiluminescent material, its fluorescence or emission spectrum is measured. When the labeling compound is a radioactive isotope, its radioactivity is measured. The signal thus measured has an indirect relation to the amount of anti-EBNA antibodies contained in the immune complex.

A measuring kit (part 1) of a second aspect of the present invention useful for the formation of the immune complex represented in Fig. 2(A) comprises the aforementioned antibody a-1, recombinant type EBNA fusion protein n and antibody b-1 labeled with a labeling compound wherein:

said antibody a-1 is immobilized to a support, said EBNA fusion protein n is bound to said antibody a-1 on said support and said labeled antibody b-1 is set to said support; or

said antibody a-1 is bound to a support, said EBNA fusion protein n is bound to said antibody a-1 thereby forming a solid phase on said support and said labeled antibody b-1 is separately included in a container.

Of these two types of the measuring kit (part 1), the former type can find assay results only by adding a test sample and measuring signals generated from the labeling compound. When the latter type is used, the results can be obtained by adding a test sample to the support, adding to the resulting support a buffer solution or the like in which the labeled antibody b-1 has been dissolved and then measuring signals generated from the labeling compound. As an alternative case of the latter type, a test sample may be added to a container containing the labeled antibody b-1, followed by the addition of the resulting mixture to the support and subsequent measurement of signals generated from the labeling compound.

A measuring kit (part 2) of the second aspect of the present invention useful for the formation of the immune complex represented in Fig. 2(A) comprises two types:

a type in which the aforementioned antibody a-1 is immobilized on a support, and the recombinant type EBNA fusion protein n and the antibody b-1 labeled with a labeling compound are adsorbed to the resulting support; and

a type in which the antibody a-1 is immobilized on a support, and the recombinant type EBNA fusion protein n and the antibody b-1 labeled with a labeling compound are included in a container.

In the latter type, the EBNA fusion protein n and the labeled antibody b-1 may be included in separate containers or in a single container.

Of these two types of the measuring kit (part 2), the former type can find assay results only by adding a test sample and measuring signals generated from the labeling compound. When the latter type is used, the results can be obtained by dissolving the EBNA fusion protein n and the labeled antibody b-1 in a test sample simultaneously or separately, adding the solution or separate solutions to the support and then measuring signals generated from the labeling compound. In the latter type, the results can be obtained also by employing additional steps in which a buffer solution and the like are used.

Measuring kits (part 3) and (part 4) of the second aspect of the present invention useful for the formation of the immune complex shown in Fig. 2(B) may be understood easily when the antibody a-1 and the EBNA fusion protein n described above in relation to the immune complex of Fig. 2(A) are replaced by an antibody a-2 and an EBNA protein (or an analog thereof) f, respectively.

In the measuring kits (part 1 to part 4), portions on the support where the immune reaction does not take place may desirably be blocked with gelatin, albumin, skim milk or the like.

Fig. 3(A) represents an immune complex which is basically the same as the immune complex of Fig. 2-(A) except that the labeled antibody b-1 is replaced by a labeled recombinant type EBNA fusion protein y-1, and Fig. 3(B) represents an immune complex which is basically the same as the immune complex of Fig. 2-(B) except that the labeled antibody b-1 is replaced by a labeled recombinant type EBNA protein (or an analog thereof) y-2.

In this instance, the labeled recombinant type EBNA fusion protein y-1 of Fig. 3(A) may be exchanged with the labeled recombinant type EBNA protein (or an analog thereof) y-2 of Fig. 3(B).

In Figs. 3(A) and 3(B), the anti-EBNA antibody s-2 to be measured in a test sample is possessed of two (or more) antigen binding sites. In other words, the immune complex shown in Fig. 3(A) is not formed when anti-EBNA antibodies to be measured are monovanent antibodies such as Fab and Fab' fragments.

More particularly, in Fig. 3(A), the anti-EBNA antibody s-2 recognizes antigen epitopes on an EBNA protein g of an EBNA fusion protein x-1 which contains the EBNA protein g and a carrier protein h and also the same epitopes on the EBNA protein g of a labeled EBNA fusion protein y-1. In Fig. 3(B), the anti-EBNA antibody s-2 recognizes epitopes on an EBNA protein (or an analog thereof) x-2 and also the same epitopes on a labeled EBNA protein y-2.

In consequence, in Fig. 3(A), the EBNA protein moiety of the EBNA fusion protein x-1 and the EBNA protein moiety of the labeled EBNA fusion protein y-1 may be different from each other, provided that both protein moieties have the same epitopes.

Also, in Fig. 3(B), the EBNA protein x-2 as a EBNA-based protein and the EBNA protein y-2, though labeled, may be different from each other, provided that both protein moieties have the same epitopes. In addtion, the EBNA protein y-2 may be an EBNA protein (or an analog thereof) having one epitope.

Since the immune complexes shown in Figs. 3(A) and 3(B) are basically the same as the immune complexes shown in Figs. 2(A) and 2(B) except for the difference in the types of labeled proteins as has been described in the foregoing, other measuring methods and kits can be designed using these immune complexes basically in the same manner by replacing the labeled antibody b-1 described in relation to Figs. 2(A) and 2(B) with the labeled recombinant type EBNA fusion protein y-1 or the labeled recombinant type EBNA protein y-2. Typical examples of such measuring methods and kits are described in the following.

Measuring method (part 5) or (part 6) in which the immune complex shown in Fig. 3(A) is formed comprises the steps of:

allowing an anti-EBNA antibody s-2 in a test sample to react with a recombinant type EBNA fusion protein x-1 (including an EBNA protein g and a carrier protein h) made into a solid phase on a support through an antibody a-1 as an anti-carrier protein antibody, allowing said anti-EBNA antibody s-2 to react with a recombinant type EBNA fusion protein y-1 (including the EBNA protein g and a carrier protein i) which has been labeled with a labeling compound, and subsequently measuring a signal generated from a labeling compound contained in the resulting immune complex which comprises the solid phase antibody a-1, the recombinant type EBNA fusion protein x-1, the anti-EBNA antibody s-2 and the labeled recombinant type EBNA fusion protein y-1 in that order; or

allowing the antibody a-1 made into a solid phase on a support to react with the recombinant type EBNA fusion protein x-1, allowing the anti-EBNA antibody s-2 in a test sample to react with the EBNA fusion protein x-1, allowing the anti-EBNA antibody s-2 to react with the recombinant type EBNA fusion protein y-1 which has been labeled with a labeling compound, and subsequently measuring a signal generated from a labeling compound contained in the resulting immune complex which comprises the solid phase antibody a-1, the recombinant type EBNA fusion protein x-1, the anti-EBNA antibody s-2 and the labeled recombinant type EBNA fusion protein y-1 in that order.

A measuring kit (part 5) in which the immune complex shown in Fig. 3(A) is formed comprises an antibody a-1, a recombinant type EBNA fusion protein x-1 and a recombinant type EBNA fusion protein y-1 which has been labeled with a labeling agent wherein:

said antibody a-1 is immobilized on a support, said EBNA fusion protein x-1 is bound to said antibody a-1 on said support and said labeled EBNA fusion protein y-1 is adsorbed to said support; or

said antibody a-1 is immobilized on a support, said EBNA fusion protein x-1 is bound to said antibody a-1 on said support and said labeled EBNA fusion protein y-1 is separately included in a container.

A measuring kit (part 6) in which the immune complex shown in Fig. 3(A) is formed comprises two types:

a type in which the antibody a-1 is bound to a support to form a solid phase, and the recombinant type EBNA fusion protein x-1 and the recombinant type EBNA fusion protein y-1 which has been labeled with a labeling compound are adsorbed to the resulting support; and

a type in which the antibody a-1 is immobilized on a support, and the recombinant type EBNA fusion protein x and the recombinant type EBNA fusion protein y-1 which has been labeled with a labeling compound are included in a container.

Measuring methods (part 7) and (part 8) of the first aspect of the present invention in which the immune complex shown in Fig. 3(B) is formed and measuring kits (part 7) and (part 8) of the second aspect of the present invention useful for the formation of said immune complex may be designed by replacing the antibody a-1 described above in relation to the immune complex of Fig. 3(A) with an antibody a-2, the EBNA fusion protein x-1 with an EBNA protein (or an analog thereof) x-2 and the labeled EBNA fusion protein y-1 with a labeled EBNA protein (or an analog thereof) y-2.

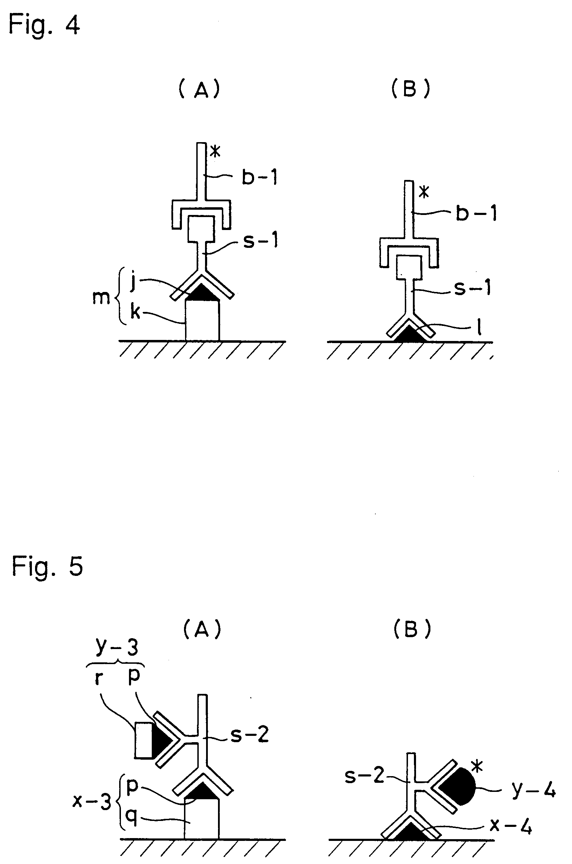

The immune complex shown in Fig. 4(A) or 4(B) is obtained by removing the antibody a-1 or the antibody a-2 from the immune complex shown in Fig. 2(A) or 2(B) and forming a solid phase of a recombinant type EBNA-based protein directly on a support.

A measuring method (part 9) of the first aspect of the present invention in which the immune complex shown in Fig. 4(A) is formed comprises the steps of:

allowing an anti-EBNA antibody s-1 in a test sample to react with a recombinant type EBNA fusion protein m (including an EBNA protein j and a carrier protein k) made into a solid phase on a support, allowing said anti-EBNA antibody s-1 to react with an antibody b-1 which has been labeled with a labeling compound, and subsequently measuring a signal generated from a labeling compound contained in the resulting immune complex which comprises the solid phase recombinant type EBNA fusion protein m, the anti-EBNA antibody s-1 and the labeled antibody b-1 in that order.

A measuring kit (part 9) of the second aspect of the present invention useful for the formation of the immune complex shown in Fig. 4(A) comprises two types:

a type in which the recombinant type EBNA fusion protein m is bound to a support to form a solid phase, and the antibody b-1 which has been labeled with a labeling compound is adsorbed to the resulting support; and

a type in which the recombinant type EBNA fusion protein m is bound to a support to form a solid phase, and the antibody b-1 which has been labeled with a labeling compound is separately included in a container.

A measuring method (part 10) of the first aspect of the present invention in which the immune complex shown in Fig. 4(B) is formed and a measuring kit (part 10) of the second aspect of the present invention useful for the formation of said immune complex may be designed by replacing the EBNA fusion protein m described above in relation to the immune complex of Fig. 4(A) with an EBNA protein (or an analog thereof) 1.

The immune complex shown in Fig. 5(A) or 5(B) is obtained by removing the antibody a-1 or the antibody a-2 from the immune complex shown in Fig. 3(A) or 3(B) and forming a solid phase of a recombinant type EBNA-based protein directly on a support.

A measuring method (part 11) of the first aspect of the present invention in which the immune complex shown in Fig. 5(A) is formed comprises the steps of:

allowing an anti-EBNA antibody s-2 in a test sample to react with a recombinant type EBNA fusion protein x-3 (including an EBNA protein p and a carrier protein q) made into a solid phase on a support, allowing said anti-EBNA antibody s-2 to react with a recombinant type EBNA fusion protein y-3 (including the EBNA protein p and a carrier protein r) which has been labeled with a labeling compound, and subsequently measuring a signal generated from a labeling compound contained in the resulting immune complex which comprises the solid phase recombinant type EBNA fusion protein x-3, the anti-EBNA antibody s-2 and the labeled recombinant type EBNA fusion protein y-3 in that order.

A measuring kit (part 11) of the second aspect of the present invention useful for the formation of the immune complex shown in Fig. 5(A) comprises two types:

a type in which the recombinant type EBNA fusion protein x-3 is bound to a support to form a solid phase and the recombinant type EBNA fusion protein y-3 which has been labeled with a labeling compound is adsorbed to the resulting support; and

a type in which the recombinant type EBNA fusion protein x-3 is immobilized on a support, and the recombinant type EBNA fusion protein y-3 which has been labeled with a labeling compound is included in a container.

A measuring method (part 12) of the first aspect of the present invention in which the immune complex shown in Fig. 5(B) is formed and a measuring kit (part 12) of the second aspect of the present invention useful for the formation of said immune complex may be designed by replacing the EBNA fusion protein x-3 described above in relation to the immune complex of Fig. 5(A) with an EBNA protein (or an analog thereof) x-4 and the labeled EBNA fusion protein y-3 with a labeled EBNA protein (or an analog thereof) y-4.

As has been described in the foregoing in relation to Figs. 3(A) and 3(B), the labeled recombinant type EBNA fusion protein y-3 of Fig. 5(A) may be exchanged with the labeled recombinant type EBNA protein (or an analog thereof) y-4 of Fig. 5(B).

In Fig. 5(A), the EBNA protein moiety of the EBNA fusion protein x-3 and the EBNA protein moiety of the labeled EBNA fusion protein y-3 may be different from each other, provided that both protein moieties have the same epitopes.

Also, in Fig. 5(B), the EBNA protein x-4 as a EBNA-based protein and the EBNA protein y-4, though labeled, may be different from each other, provided that both protein moieties have the same epitopes. In addition, the EBNA protein y-4 may be an EBNA protein (or an analog thereof) having one epitope.

In the measuring methods and measuring kits of the first and second aspects of the present invention in which the immune complexes shown in Figs. 3(A), 3(B), 4(A), 4(B), 5(A) and 5(B) are used, optimum immune reaction conditions, blocking treatment and other necessary conditions are basically the same as those described in the foregoing in relation to Figs. 2(A) and 2(B).

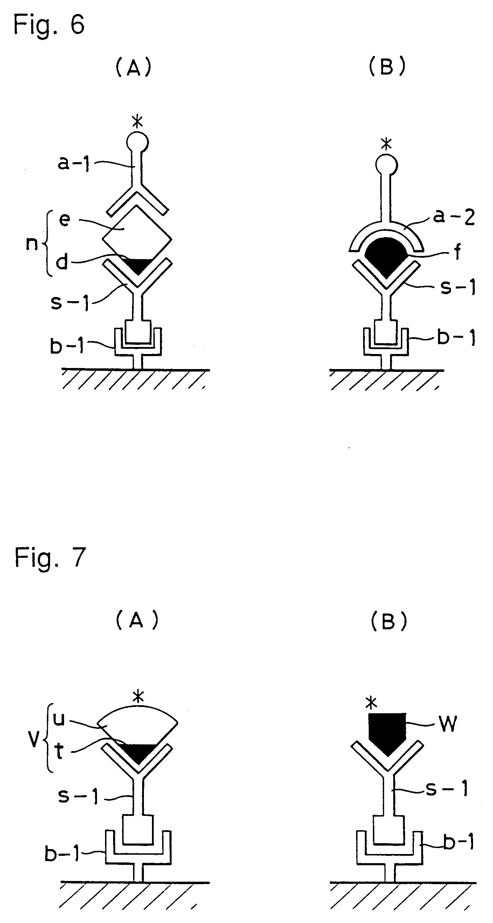

Fig. 6(A) is a pictorial view showing an immune complex comprising a solid phase antibody b-1, an anti-EBNA antibody s-1, a recombinant type EBNA fusion protein n and a labeled antibody a-1 in that order, which is formed through the steps of; allowing the antibody b-1 immobilized on a support to react with the anti-EBNA antibody s-1 in a test sample, allowing said anti-EBNA antibody s-1 to react with the recombinant type EBNA fusion protein n (including an EBNA protein d and a carrier protein e) and then allowing an anti-carrier protein antibody labeled with a labeling compound, namely the labeled antibody a-1, to react with the recombinant type EBNA fusion protein n. Fig. 6(B) is a pictorial view showing an immune complex comprising a solid phase antibody b-1, an anti-EBNA antibody s-1, a recombinant type EBNA protein (or an analog thereof) f and a labeled antibody a-2 in that order, which is formed through the steps of; allowing the antibody b-1 made into a solid phase on a support to react with the anti-EBNA antibody s-1 in a test sample, allowing said anti-EBNA antibody s-1 to react with the recombinant type EBNA protein (or an analog thereof) f and then allowing an anti-EBNA antibody labeled with a labeling compound, namely the labeled antibody a-2, to react with said recombinant type EBNA protein (or an analog thereof) f.

A measuring method (part 13) of the first aspect of the present invention in which the immune complex shown in Fig. 6(A) is formed comprises the steps of; allowing an antibody b-1 made into a solid phase on a support to react with an anti-EBNA antibody s-1 in a test sample, allowing said anti-EBNA antibody s-1 to react with a recombinant type EBNA fusion protein n, allowing an antibody a-1 labeled with a labeling compound to react with said recombinant type EBNA fusion protein n, and subsequently measuring a signal generated from a labeling compound contained in the resulting immune complex which comprises the solid phase antibody b-1, the anti-EBNA antibody s-1, the recombinant type EBNA fusion protein n and the labeled antibody a-1 in that order. Illustratively, the measuring method may be effected for example by adding a test sample to the aforementioned support on which a solid phase of the antibody b-1 is made, thereby effecting binding of said antibody b-1 to the anti-EBNA antibody s-1 in the test sample, adding the EBNA fusion protein n, thereby effecting binding of the fusion protein n to the anti-EBNA antibody s-1, adding the labeled antibody a-1, thereby effecting binding of the labeled antibody a-1 to the fusion protein n, and subsequently measuring a signal generated from a labeling compound contained in the resulting immune complex.

The measuring method (part 13) includes variations in which the binding order is changed or at least two of the binding reactions are carried out at the same time. Examples of such variations to be included in the measuring method (part 13) of the first aspect of the present invention include a variation in which a complex compound prepared from the fusion protein n and the labeled antibody a-1 is used and a variation in which a complex compound consisting of the anti-EBNA antibody s-1 in a test sample, the fusion protein n and the labeled antibody a-1 is firstly formed, the resulting complex compound is bound to the solid phase antibody b-1 on a support and then the signal generated from a labeling compound is measured.

A measuring method (part 14) of the first aspect of the present invention in which the immune complex shown in Fig. 6(B) is formed may be designed by replacing the EBNA fusion protein n described above in relation to the immune complex of Fig. 6(A) with an EBNA protein (or an analog thereof) f and replacing the labeled antibody a-1 with a labeled antibody a-2.

In these measuring methods (part 13) and (part 14), optimum immune reaction conditions, measuring procedures of signals generated from a labeling compound and other necessary conditions are basically the same as those described in the foregoing in relation to Figs. 2(A) and 2(B).

A measuring kit (part 13) of the second aspect of the present invention useful for the formation of the immune complex shown in Fig. 6(A) comprises two types:

a type in which the antibody b-1 is bound to a support to form a solid phase, and the recombinant type EBNA fusion protein n and the antibody a-1 which has been labeled with a labeling compound are adsorbed to the resulting support with or without mutual bonding; and

a type in which the antibody b-1 is bound to a support to form a solid phase, and the recombinant type EBNA fusion protein n and the antibody a-1 which has been labeled with a labeling compound are included in a container with or without mutual binding.

Of these two types of the measuring kit (part 13), the former type can find assay results only by adding a test sample and measuring signals generated from the labeling compound. When the latter type is used, the results can be obtained by adding a test sample to the support, adding to the resulting support a buffer solution or the like in which the EBNA fusion protein n and the labeled antibody a-1 have been dissolved and then measuring signals generated from the labeling compound.

A measuring kit (part 14) of the second aspect of the present invention useful for the formation of the immune complex shown in Fig. 6(B) may be designed by replacing the the EBNA fusion protein n described above in relation to the immune complex of Fig. 6(A) with an EBNA protein (or an analog thereof) f and replacing the labeled antibody a-1 with a labeled antibody a-2.

In these measuring kits (part 13) and (part 14) of the second aspect of the present invention, blocking treatment and other necessary conditions are basically the same as those described in the foregoing in relation to Figs. 2(A) and 2(B).

Fig. 7(A) represents an immune complex which is basically the same as the immune complex of Fig. 6-(A) except that the recombinant type EBNA fusion protein n and the labeled antibody a-1 are replaced by a single substance which can undergo functions of these two compounds simultaneously, that is, a labeled recombinant type EBNA fusion protein v (containing an EBNA protein t and a carrier protein u), and Fig. 7-(B) represents an immune complex which is basically the same as the immune complex of Fig. 6(B) except that the recombinant type EBNA protein (or an analog thereof) f and the labeled antibody a-2 are replaced by a labeled recombinant type EBNA protein w. In this instance, the labeled recombinant type EBNA fusion protein v of Fig. 7(A) and the labeled recombinant type EBNA protein (or an analog thereof) w of Fig. 7(B) are exchangeable.

A measuring method (part 15) of the first aspect of the present invention in which the immune complex shown in Fig. 7(A) is formed comprises the steps of; allowing an anti-EBNA antibody s-1 in a test sample to react with an antibody b-1 immobilized on a support, allowing said anti-EBNA antibody s-1 to react with a recombinant type EBNA fusion protein v (including an EBNA protein t and a carrier protein u) which has been labeled with a labeling compound, and subsequently measuring a signal generated from a labeling compound contained in the resulting immune complex which comprises the solid phase antibody b-1, the anti-EBNA antibody s-1 and the labeled recombinant type EBNA fusion protein v in that order.

A measuring kit (part 15) of the second aspect of the present invention in which the immune complex shown in Fig. 7(A) is formed comprises two types:

a type in which the antibody b-1 is immobilized on a support, and the recombinant type EBNA fusion protein v which has been labeled with a labeling compound is adsorbed to the resulting support; and

a type in which the antibody b-1 is immobilized on a support, and the recombinant type EBNA fusion protein v which has been labeled with a labeling compound is separately included in a container.

A measuring method (part 16) of the first aspect of the present invention in which the immune complex shown in Fig. 7(B) is formed and a measuring kit (part 16) of the second aspect of the present invention useful for the formation of said immune complex may be designed by replacing the labeled EBNA fusion protein v described above in relation to the immune complex of Fig. 7(A) with a labeled EBNA protein w.

In these measuring methods (part 15) and (part 16) and measuring kits (part 15) and (part 16) of the first and second aspect of the present invention, optimum immune reaction conditions, blocking treatment and other necessary conditions are basically the same as those described in the foregoing in relation to Figs. 2(A) and 2(B).

In the measuring methods (part 13) to (part 16) in which the immune complexes shown in Figs. 6(A), 6-(B), 7(A) and 7(B) are formed, a washing step which is useful for removing the influence of non-specific immunoglobulins in a test sample cannot be employed, though these methods are useful for the measurement of low blood level class and subclass anti-EBNA antibodies such as IgM, IgA and the like.

Thus, various examples in which the measurement is based on the sandwich method have been described

Next, other examples in which the measurement is based on the competitive binding method are described.

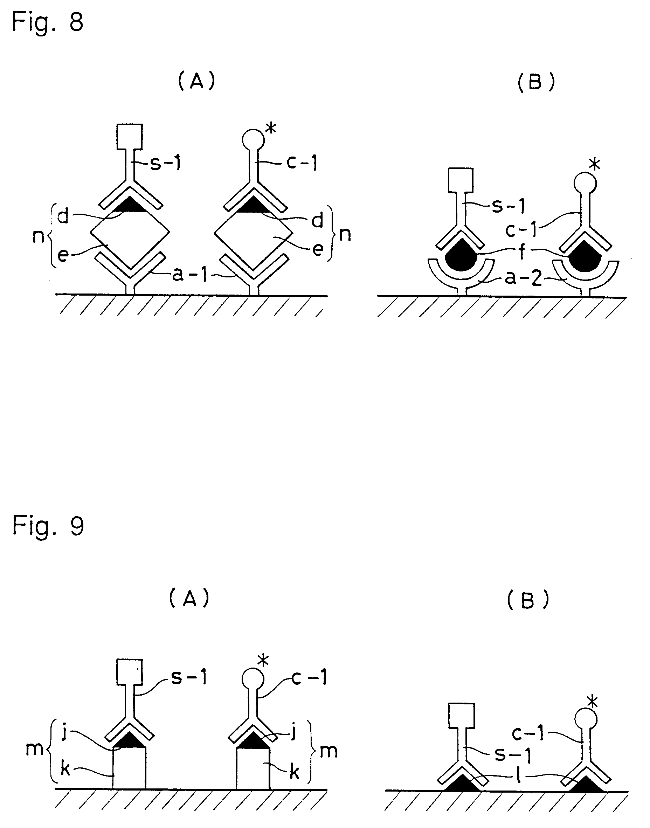

Fig. 8(A) is a pictorial view showing an immune complex comprising a solid phase antibody a-1, a recombinant type EBNA fusion protein n and an anti-EBNA antibody s-1 in that order (the left side picture in the figure) and another immune complex comprising the solid phase antibody a-1, the recombinant type EBNA fusion protein n and a labeled antibody c-1 in that order (the right side picture in the figure), which are formed through the step or steps of:

allowing the recombinant type EBNA fusion protein n (containing an EBNA protein d and a carrier protein e) immobilized on a support through the antibody a-1 as an anti-carrier protein antibody to undergo competitive reaction with the anti-EBNA antibody s-1 in a test sample and the labeled antibody c-1 as an anti-EBNA antibody labeled with a labeling compound; or

allowing the antibody a-1 immobilized on a support to react with the EBNA fusion protein n, and subsequently allowing the EBNA fusion protein n to undergo competitive reaction with the anti-EBNA antibody s-1 in a test sample and the labeled antibody c-1.

Fig. 8(B) is a pictorial view showing an immune complex comprising a solid phase antibody a-2, a recombinant type EBNA protein f and an anti-EBNA antibody s-1 in that order (the left side picture in the figure) and another immune complex comprising the solid phase antibody a-2, the recombinant type EBNA protein f and a labeled antibody c-1 in that order (the right side picture in the figure), which are formed through the step or steps of:

allowing the recombinant type EBNA protein (or an analog thereof) f immobilized on a support through the antibody a-2 as an anti-EBNA antibody to undergo competitive reaction with the anti-EBNA antibody s-1 in a test sample and the labeled antibody c-1 as an anti-EBNA antibody labeled with a labeling compound; or

allowing the antibody a-2 immobilized on a support to react with the EBNA protein (or an analog thereof) f, and subsequently allowing the EBNA protein f to undergo competitive reaction with the anti-EBNA antibody s-1 in a test sample and the labeled antibody c-1.

A measuring method (part 17) of the first aspect of the present invention in which the immune complex shown in Fig. 8(A) is formed comprises the steps of; allowing the recombinant type EBNA fusion protein n immobilized on a support through the antibody a-1 to undergo competitive reaction with the anti-EBNA antibody s-1 in a test sample and the antibody c-1 labeled with a labeling compound, thereby forming an immune complex comprising the solid phase antibody a-1, the recombinant type EBNA fusion protein n and the anti-EBNA antibody s-1 in that order and another immune complex comprising the solid phase antibody a-1, the recombinant type EBNA fusion protein n and the labeled antibody c-1 in that order, and subsequently measuring signals generated from a labeling compound in the immune complex containing the labeled antibody c-1. Illustratively, the measuring method is effected for example by adding a test sample and the labeled antibody c-1 simultaneously or by turns to the support on which the recombinant type EBNA fusion protein n has been made into a solid phase through the antibody a-1, thereby allowing the EBNA fusion protein n to undergo competitive reaction with the anti-EBNA antibody s-1 in the test sample and the labeled antibody c-1, and then measuring signals generated from one of the thus formed two immune complexes shown in Fig. 8(A), the one which contains the labeled antibody c-1.

A measuring method (part 18) of the first aspect of the present invention in which the immune complex shown in Fig. 8(A) is formed comprises the steps of; allowing the antibody a-1 immobilized on a support to react with the recombinant type EBNA fusion protein n and allowing the recombinant type EBNA fusion protein n to undergo competitive reaction with the anti-EBNA antibody s-1 in a test sample and the antibody c-1 labeled with a labeling compound, thereby forming an immune complex comprising the solid phase antibody a-1, the recombinant type EBNA fusion protein n and the anti-EBNA antibody s-1 in that order and another immune complex comprising the solid phase antibody a-1, the recombinant type EBNA fusion protein n and the labeled antibody c-1 in that order, and subsequently measuring signals generated from a labeling compound in the immune complex containing the labeled antibody c-1. Illustratively, the measuring method is effected for example by adding the EBNA fusion protein n to the support on which the antibody a-1 has been made into a solid phase, thereby effecting binding of the antibody a-1 to the EBNA fusion protein n, and then adding a test sample and the labeled antibody c-1 simultaneously or by turns, thereby allowing the EBNA fusion protein n to undergo competitive reaction with the anti-EBNA antibody s-1 in the test sample and the labeled antibody c-1, and subsequently measuring signals generated from one of the thus formed two immune complexes shown in Fig. 8(A), the one which contains the labeled antibody c-1.

The measuring method (part 18) includes variations in which the binding order is changed or at least two of the binding reactions are carried out at the same time. Examples of such variations to be included in the measuring method (part 18) of the first aspect of the present invention include a method in which the anti-EBNA antibody s-1 in a test sample is allowed to bind to the EBNA fusion protein n in competition with the labeled antibody c-1 to form two types of immune complexes, the thus formed two immune complexes are allowed to bind to the solid phase antibody a-1 on a support and then the signal generated from a labeling compound is measured.

Measuring methods (part 19) and (part 20) of the first aspect of the present invention in which the immune complex shown in Fig. 8(B) is formed may be designed by replacing the antibody a-1 described above in relation to the immune complex of Fig. 8(A) with an anti-EBNA antibody, namely an antibody a-2, and replacing the EBNA fusion protein n with an EBNA protein (or an analog thereof) f.

If necessary, a washing step making use of a buffer solution or the like may be added to each of these measuring methods. Examples of such buffer solutions and related conditions, as well as immune reaction conditions and signal-measuring procedure, have already been described in the foregoing with respect to the sandwich-based measuring methods of the first aspect of the present invention.

A measuring kit (part 17) of the second aspect of the present invention useful for the formation of the immune complex shown in Fig. 8(A) comprises two types:

a type in which the antibody a-1 is immobilized on a support, the recombinant type EBNA fusion protein n is bound to the antibody a-1 on the support, and the antibody c-1 which has been labeled with a labeling compound is adsorbed to the support or bound to the EBNA fusion protein n; and

a type in which the antibody a-1 is immobilized on a support, the recombinant type EBNA fusion protein n is bound to the antibody a-1 on the support, and the antibody c-1 which has been labeled with a labeling compound is separately included in a container.

Of these two types of the measuring kit (part 17), the former type can find assay results only by adding a test sample and measuring signals generated from the labeling compound. When the latter type is used, the results can be obtained by adding a test sample to a container which has been charged with the labeled antibody c-1, adding the resulting mixture of the labeled antibody c-1 with the sample to the support, and then measuring signals generated from the labeling compound.

A measuring kit (part 18) of the second aspect of the present invention useful for the formation of the immune complex shown in Fig. 8(A) comprises two types:

a type in which the antibody a-1 is immobilized on a support, and the recombinant type EBNA fusion protein n and the antibody c-1 which has been labeled with a labeling compound are adsorbed, with or without mutual binding, to the support; and

a type in which the antibody a-1 is immobilized on a support, and the recombinant type EBNA fusion protein n and the antibody c-1 which has been labeled with a labeling compound are included in a container with or without mutual binding.

Of these two types of the measuring kit (part 18), the former type can find assay results only by adding a test sample and measuring signals generated from the labeling compound. When the latter type is used, the results can be obtained by dissolving the EBNA fusion protein n and the labeled antibody c-1 in portions of a test sample, adding the resulting solutions to the support simultaneously or by turns, and then measuring signals generated from the labeling compound. In the case of the latter type, the results can also be obtained by employing additional steps such as a washing step in which a buffer solution or the like is used.

Measuring kits (part 19) and (part 20) of the second aspect of the present invention useful for the formation of the immune complex shown in Fig. 8(B) may be designed by replacing the antibody a-1 described above in relation to the immune complex of Fig. 8(A) with an anti-EBNA antibody a-2, and replacing the EBNA fusion protein n with an EBNA protein (or an analog thereof) f.

The immune complex shown in Fig. 9(A) or 9(B) is obtained by removing the antibody a-1 or the antibody a-2 from the immune complex shown in Fig. 8(A) or 8(B) and forming a solid phase of a recombinant type EBNA-based protein directly on a support.

A measuring method (part 21) of the first aspect of the present invention in which the immune complex shown in Fig. 9(A) is formed comprises the steps of; allowing a recombinant type EBNA fusion protein m (containing an EBNA protein j and a carrier protein k) immobilized on a support to undergo competitive reaction with an anti-EBNA antibody s-1 in a test sample and an antibody c-1 labeled with a labeling compound, thereby forming an immune complex comprising the solid phase recombinant type EBNA fusion protein m and the anti-EBNA antibody s-1 in that order (the left side picture in Fig. 9(A)) and another immune complex comprising the solid phase recombinant type EBNA fusion protein m and the labeled antibody c-1 in that order (the right side picture in the figure), and subsequently measuring signals generated from a labeling compound in the immune complex containing the labeled antibody c-1.

A measuring kit (part 21) of the second aspect of the present invention useful for the formation of the immune complex shown in Fig. 9(A) comprises two types:

a type in which the recombinant type EBNA fusion protein m is immobilized on a support, and the antibody c-1 which has been labeled with a labeling compound is adsorbed to the support or bound to the EBNA fusion protein m; and

a type in which the recombinant type EBNA fusion protein m immobilized on the support, and the antibody c-1 which has been labeled with a labeling compound is separately included in a container.

EP 0 508 427 A2

A measuring method (part 22) of the first aspect of the present invention in which the immune complex shown in Fig. 9(B) is formed and a measuring kit (part 22) of the second aspect of the present invention useful for the formation of said immune complex may be designed by replacing the EBNA fusion protein m described above in relation to the immune complex of Fig. 9(A) with an EBNA protein (or an analog thereof) 1.

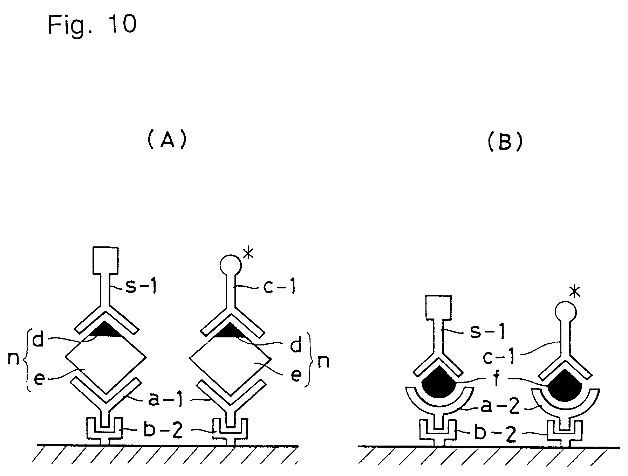

The immune complex shown in Fig. 10(A) or 10(B) is obtained by forming a solid phase of the immune complex of Fig. 8(A) or 8(B) on a support through the antibody a-2 as an anti-antibody.

Measuring methods (part 23), (part 24) and (part 25) of the first aspect of the present invention in which the immune complex shown in Fig. 10(A) is formed comprises the steps of:

allowing a recombinant type EBNA fusion protein n (containing an EBNA protein d and a carrier protein e) immobilized on a support through an antibody b-2 and an anti-carrier protein antibody a-1 to undergo competitive reaction with an anti-EBNA antibody s-1 in a test sample and an anti-EBNA antibody c-1 which has been labeled with a labeling compound, or

allowing the antibody a-1 immobilized on a support through the antibody b-2 to react with the EBNA fusion protein n and allowing the EBNA fusion protein n to undergo competitive reaction with the anti-EBNA antibody s-1 in a test sample and the antibody c-1 which has been labeled with a labeling compound, or

allowing the antibody b-2 immobilized on a support to react with the antibody a-1, allowing the EBNA fusion protein n to react with the antibody a-1 and then allowing the EBNA fusion protein n to undergo competitive reaction with the anti-EBNA antibody s-1 in a test sample and the antibody c-1 which has been labeled with a labeling compound,

thereby forming an immune complex comprising the solid phase antibody b-2, the antibody a-1, the recombinant type EBNA fusion protein n and the anti-EBNA antibody s-1 in that order (the left side picture in Fig. 10(A)) and another immune complex comprising the solid phase antibody b-2, the antibody a-1, the recombinant type EBNA fusion protein n and the labeled antibody c-1 in that order (the right side picture in the figure), and

measuring signals generated from a labeling compound in the immune complex containing the labeled antibody c-1.

A measuring kit (part 23) of the second aspect of the present invention useful for the formation of the immune complex shown in Fig. 10(A) comprises the aforementioned antibody b-2, antibody a-1 and EBNA fusion protein n wherein:

the antibody b-2 is immobilized on a support, the antibody a-1 is bound to the antibody b-2 and the EBNA fusion protein n is bound to the antibody a-1 to form a solid phase on the support, and the antibody c-1 which has been labeled with a labeling compound is set to the support or bound to the EBNA fusion protein n, or

the antibody b-2 is immobilized on a support, the antibody a-1 is bound to the antibody b-2 and the EBNA fusion protein n is bound to the antibody a-1 to form a solid phase on the support, and the antibody c-1 which has been labeled with a labeling compound is separately included in a container.

A measuring kit (part 24) of the second aspect of the present invention useful for the formation of the immune complex shown in Fig. 10(A) comprises two types:

a type in which the antibody b-2 is bound to a support and the antibody a-1 is bound to the antibody b-2 to form a solid phase on the support, and the EBNA fusion protein n and the antibody c-1 which has been labeled with a labeling compound are adsorbed, with or without mutual bonding, to the support, and

a type in which the antibody b-2 is immobilized on a support and the antibody a-1 is bound to the antibody b-2 to form a solid phase on the support, and the EBNA fusion protein n and the antibody c-1 which has been labeled with a labeling compound are separately included in a container with or without mutual binding.

A measuring kit (part 25) of the second aspect of the present invention useful for the formation of the immune complex shown in Fig. 10(A) comprises two types:

a type in which the antibody b-2 is immobilized on a support, and the antibody a-1, the EBNA fusion protein n and the antibody c-1 which has been labeled with a labeling compound are set, with or without mutual bonding, to the support, and

a type in which the antibody b-2 is immobilized on a support, and the antibody a-1, the EBNA fusion protein n and the antibody c-1 which has been labeled with a labeling compound are separately included in a container with or without mutual binding.

In this instance, each of the measuring kits (part 24) and (part 25) includes a variation in which, among two or three composing elements that are not made into solid phases on a support, one or two of them are set on the support and other one or two are separately included in a container.

Measuring methods (part 26), (part 27) and (part 28) of the first aspect of the present invention in which the immune complex shown in Fig. 10(B) is formed and measuring kits (part 26), (part 27) and (part 28) of the second aspect of the present invention useful for the formation of said immune complex may be designed by replacing the antibody a-1 described above with respect to the immune complex of Fig. 10(A) with an anti-EBNA antibody a-2, and the EBNA fusion protein n with an EBNA protein (or an analog thereof) f.

In these measuring methods (part 21 to part 28) and measuring kits (part 21 to part 28) of the first and second aspects of the present invention in which the immune complexes shown in Figs. 9(A), 9(B), 10(A) and 10(B) are formed, optimum immune reaction conditions, blocking treatment and other necessary conditions are basically the same as those described in the foregoing with respect to Fig. 8(A).

The following describes a process for the production of the measuring kit of the second aspect of the present invention.

Though not particularly limited, the measuring kit of the second aspect of the present invention may be a vertical type in which a liquid added to a support flows in vertical direction or a chromatographic type in which a certain material such as a strip is used as a support and an added liquid flows in horizontal direction.

In either case, proteinous substances such as antibodies are immobilized on a support.

Formation of a solid phase of the antibody a, the antibody b or the recombinant type EBNA-based protein on a support may be effected in accordance with known means such as the procedure disclosed in *Enzyme Immuoassay* (Ishikawa *et al.*, described in the foregoing).

After making at least one of the antibody a, the antibody b and the recombinant type EBNA-based protein into a solid phase on a support, the resulting support is washed to remove nonbound portions. When at least two substances are made into a solid phase, an additional washing step may be employed between the binding reactions.

The thus washed support may be subjected to aftercoating making use of an appropriate protein material. Such a treatment is employed to protect blank spaces on the support (where the antibody a or the EBNA-based protein is not bound) from non-specific adsorption of various materials which are used in the subsequent steps.

Any protein source may be selected for use in the aftercoating, provided that it has the just described function. Preferred examples of such protein sources include skim milk, BSA (bovine serum albumin) and the like. Aftercoating may be carried out for a certain period of time which is sufficient enough to complete coating of a support, but preferably at a low temperature for a prolonged period of time in order to prevent denaturation of protein, more particularly at 4°C for at least an overnight length.

When the aftercoating is completed, the thus treated support is washed to remove excessive substances remained on the support.

In the case of the production of a measuring kit containing a means in which a labeled antibody c is attached to the aforementioned EBNA-based protein (made into a solid phase on the support directly or through the aforementioned antibody a), the substance to be attached is added to the resulting washed support and incubated under proper conditions, preferably at room temperature for several hours, more preferably at 37°C for 1 hour. After completion of the incubation, the resulting support is washed to remove unreacted substances.

In this way, a support preparation is obtained in which at least one of the antibody a, the antibody b and the recombinant type EBNA-based protein is immobilized on a support, or a support in which other substances are further attached to the solid phase.

Other composing elements of the measuring kit of the second aspect of the present invention which cannot be combined directly or indirectly may be attached to the support in the form of freeze-dried powder or provided by separately enclosing in a container.

Next, a third aspect of the present invention is described.

According to the third aspect of the present invention, there is provided a panel type anti-EBNA antibody measuring method which can measure at least two different types of anti-EBNA antibodies in one test sample simultaneously and individually.

The panel type method which can measure at least two different types of anti-EBNA antibodies in one test sample simultaneously and individually. In consequence, antibody titers against each EBNA polypeptide can be obtained simultaneously, individually and accurately using by this panel type method.

Antibody titers against each EBNA polypeptide can render possible more detailed analysis of diseases under suspicion of EBV-infection. In addition, such a measuring method will find versatile use in such applications as definite diagnosis of a disease, understanding of the stage of a disease and prevention of a disease.

In this panel type measuring method of the present invention, the following panel type kit is preferably used.

According to the third aspect of the present invention, there is provided a panel type anti-EBNA antibody measuring kit which can measure at least two different types of anti-EBNA antibodies in one test sample simultaneously and individually. Particularly, the third aspect of the present invention provides a panel type anti-EBNA antibody measuring kit according to the aforementioned measuring kit of the second aspect of the present invention wherein at least two recombinant type EBNA-based proteins are used which are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein and a recombinant type EBNA-6-based protein, and antibodies specific for the selected protein antigens can be measured simultaneously.

In the measuring kit of the third aspect of the present invention, each of the recombinant type EBNA-based proteins is arranged separately on a support and/or separately included in a container. For example, when two types of EBNA-based proteins x and y are used as the recombinant type EBNA-based proteins, the EBNA-based proteins x and y may be arranged on a support as a combination, or x on the support and y in a container.

In this instance, the term "a recombinant type EBNA-based protein arranged on a support" as used herein means a state in which the protein is immobilized directly on the support, made into a solid phase through the aforementioned antibody a, antibody b and the like, or set on the support.

In the case of such a panel type anti-EBNA antibody measuring kit, it is preferable to use fusion proteins as the recombinant type EBNA-based proteins and to form directly on a support a solid phase of the antibody a which is the antibody specific for the carrier protein moiety of the fusion protein.

In the case of such a type of the measuring kit, a support may have a solid phase of only the antibody a. In that instance, the measuring kit may have an advantage in that the support having a single solid phase of the antibody a can be used independent of the types of anti-EBNA antibodies to be measured, because any of the fusion proteins including an EBNA-1 fusion protein, an EBNA-2 fusion protein, an EBNA-3 fusion protein, an EBNA-4 fusion protein, an EBNA-5 fusion protein and an EBNA-6 fusion protein can be bound to the solid phase antibody a through the carrier protein moiety of each fusion protein.

In the panel type anti-EBNA antibody measuring kit of the third aspect of the present invention which is based on the sandwich method, it is preferable to use, as the labeled antibody b, anti-human immunoglobulin antibodies to which an appropriate labeling compound is attached. By the use of such a type of the measuring kit, any type of the anti-EBNA antibodies including the anti-EBNA-1 antibody, the anti-EBNA-2 antibody, the anti-EBNA-3 antibody, the anti-EBNA-4 antibody, the anti-EBNA-5 antibody and the anti-EBNA-6 antibody can be measured with only one type of the labeled antibody b, because anti-human immunoglobulin antibodies react evenly with these 6 types of antibodies in a test sample.

In the case of such a measuring kit in which labeled anti-human immunoglobulin antibodies are used as the labeled antibody b, it is desirable to employ a washing step prior to the reaction step of anti-EBNA antibodies with the labeled anti-human immunoglobulin antibodies in order to remove unreacted components in a test sample.

EXAMPLES

The following examples are provided to further illustrate the present invention. It is to be understood, however, that the examples are for purpose of illustration only and are not intended as a definition of the limits of the invention.

(Example 1) Isolation of EBNA subfragments

A *Bam*HI fragment of the EBV-DNA in an EBV-positive cell line B95-8 was digested with various appropriate restriction enzymes in the usual way to isolate each subfragment shown in Table B and Fig. 1.

Restriction enzymes used for the isolation of each subfragment are shown in Fig. 1 in which each enzyme is expressed by the following abbreviations.

Aa, *Aat*II; Al, *Alu*I; Bl, *Bal*I; Bm, *Bam*HI; Dr, *Dra*I; ER, *Eco*RI; EV*, Eco*RV; Hi, *Hind*III; Hp, *Hpa*I, Nc, *Nco*I; Pv, *Pvu*II; SII, *Sac*II; Sm, *Sma*I; Sp, *Sph*I

(Example 2) Preparation of *β*-galactosidase-EBNA fusion protein

(1) Construction of vector

Each of the subfragments isolated in Example 1 was repaired with Klenow fragment of DNA polymerase I, linked to appropriate lengths of *Eco*RI linkers (Pharmacia) and then inserted into an EcoRI site of λgt11 (Young, R.A. *et al.*, Proc. Natl. Acad. Sci., USA, 80, pp.1194 - 1198, 1983) DNA. Since the *Eco*RI site locates close to the 3'-end of β-galactosidase gene in λgt11, each of the isolated fragments becomes a fused form with the β-galactosidase gene. The λgt11 DNA contains cl857 as a λ phage repressor gene. Recombinant DNA fragments thus obtained by introducing respective subfragments together with the *Eco*RI linkers were subjected to phage packaging in accordance with the method of Hohn *et al.* (Methods in Enzymology, 68, pp.299 - 309, 1979) to obtain vectors (recombinant phages).

(2) Preparation of transformant

An *E. coli* strain Y1089 (Young, R.A. and Devis, R.W., Science, 222, pp.778 - 782, 1983) was lysogenized with each of the recombinant phages prepared in Example 2-(1) to isolate lysogenic strains in the usual way.

(3) Culturing of transformant and recovery of fusion protein

Each of the lysogenic *E. coli* strains prepared in Example 2-(2) was cultured at 32°C for 2 to 3 hours on a shaker using 0.85 liter of TYλ medium (10 g of Bacto-trypton, 1 g of yeast extracts and 2.5 g of NaCl in one liter of water) which has been supplemented with 40 μg/ml of ampicillin and 10 μg/ml of tetracycline.

When the strain grew into a cell density of $1 \times 10^6$ cells/ml, the culture temperature was increased to 45°C. After 20 minutes of shaking culture at the elevated temperature, 0.1 mM of IPTG (isopropyl-1-thio-β-D-galactopyranoside) was added and the shaking culturing was continued at 39°C for 90 minutes.

The thus obtained culture broth was cooled in an ice bath for 5 minutes and then subjected to centrifugation at 8,000 x g for 5 minutes (centrifuge, model KR-20000T manufactured by Kubota Corporation).

The resulting precipitate was suspended in 10 ml of 10 mM Tris-HCl buffer (pH 7.5) containing 200 mM NaCl and 1 mM of EDTA (to be referred to as "Buffer A" hereinafter). The thus prepared cell suspension was mixed with 1 ml of lysozyme solution (10 mg/ml), allowed to stand still at 0°C for 10 to 15 minutes and then mixed with a nonionic surface active agent, CHAPSO (3[(3-colamidiopropyl)-dimethylammonio]-2-hydroxypropane sulfate, Dojindo Laboratories), to a final concentration of 0.5% (w/v).

After repeating a freeze-thawing step twice using a dry ice-methanol system, the resulting mixture was subjected to ultrasonic treatment.

The thus obtained solution by the ultrasonic treatment was subjected to ultracentrifugation at 30,000 x g for 30 minutes, and the resulting supernatant was recovered. The remaining precipitate was dissolved in 20 ml of Buffer A, and the solution was subjected to ultrasonic treatment and then to ultracentrifugation. The resulting supernatant was mixed with the first supernatant fluid obtained above to use as a crude extract.

(4) Purification of fusion protein

About 150 ml portion of each of the fusion protein-containing crude extracts obtained in Example 2-(3) was dialyzed against 20 mM Tris buffer containing 200 mM NaCl (to be referred to as "Buffer B" hereinafter).

The thus dialyzed crude extract was fractionated by salting-out techniques. That is, ammonium sulfate was added to the crude extract to a concentration of 30% saturation, and the mixture was stirred in an ice bath for 1 to 2 hours to dissolve the salt completely and then allowed to stand still overnight at 4°C.

The thus treated extract was subjected to centrifugation at 4°C and at 6,000 x g for 30 minutes. The resulting precipitate was dissolved in Buffer B, dialyzed against the same buffer and then diluted with 200 ml of the same buffer.

A portion (1 g as protein) of the diluted sample thus prepared was applied spending an overnight period to DEAE cellulose column (3-5 cmØ x 15-20 cm) which has been equllibrated with Buffer B. After washing the column with 100 to 200 ml of Buffer B, elution was effected with 20 mM Tris buffer containing 300 mM NaCl.

During the elution, absorbance at 280 nm was measured to monitor protein in the eluates which were pooled into protein peak fractions. Thereafter, concentrated fusion protein samples were obtained by salting-out techniques in the same manner as described above in which final concentration of ammonium sulfate was changed within the range of from 30 to 40% saturation depending on each pooled fraction. When required, the samples were further purified by means of affinity chromatography.

Expressed amount of each fusion protein is shown in the following table.

Table B

| EBNA | Size (a.a.) | Carrier protein | Code | Domain (a.a.) | Expression |
|---|---|---|---|---|---|
| EBNA-1 | 641 | $\beta$-galactosidase | K1 | 39 - 641 | + |
| | | | K2 | 451 - 641 | + + |
| | | | D1 | 451 - 581 | + + + |
| | | | SI | 488 - 641 | + + |
| | | | SII | 451 - 618 | + + + |
| | | | HS | 460 - 618 | + + |
| EBNA-2 | 487 | $\beta$-galactosidase | H3 | 325 - 487 | + |
| | | | H4 | 116 - 324 | + |
| | | | H5 | 116 - 246 | + |
| | | | H6 | 248 - 324 | + |
| | | | H7 | 383 - 487 | + |
| | | protein A | Y1 | 1 - 116 | + + + |
| | | | 0.5K | 147 - 323 | + |
| BERF1 (EBNA-3) | 839 | $\beta$-galactosidase | B4 | 21 - 174 | + + |
| | | | S8 | 462 - 713 | + + + |
| | | | S4 | 714 - 839 | + + + |
| BERF2b (EBNA-4) | 840 | $\beta$-galactosidase | N4 | 379 - 499 | + + + |
| | | | N5 | 215 - 379 | + + + |
| | | | HP | 679 - 840 | + + |
| BERF4 (EBNA-6) | 872 | $\beta$-galactosidase | AE | 426 - 593 | + |
| | | | A1 | 269 - 517 | + + |

The inventors of the present invention have isolated *E. coli* strains transformed with vectors containing DNA fragments coding for the EBNA fusion proteins shown in the above table. These transformants have been deposited by the present inventors on December 13, 1990, in Fermentation Research Institute, Agency of Industrial Science and Technology, 1-3, Higashi 1 chome Tsukuba-shi, Ibaraki-ken 305, Japan, and have been assigned the designations as FERM P-11902, transferred to International Deposit No. BP-3785 (expresses EBNA-6 (A1)), FERM P-11903, transferred to International Deposit No. BP-3786 (expresses EBNA-2 (H7)), FERM P-11904, transferred to International Deposit No. BP-3787 (expresses EBNA-1 (K2)), FERM P-11905, transferred to International Deposit No. BP-3788 (expresses EBNA-4 (N4)) and FERM P-11906, transferred to International Deposit No. BP-3789 (expresses EBNA-3 (S8)).

(Example 3) Production of protein containing a portion of EBNA-2

(1) Construction of vector

A region from *Rsa*I site to *Bam*HI site of the subfragment Y1 prepared in Example 1 containing a DNA fragment coding for a portion of EBNA-2 was inserted into plasmid pUC118 (Vieira, J. *et al.*, Methods Enzymol.,153, pp.3 - 11, 1987). The thus inserted plasmid was subjected to trimming using *Bal*31 exonuclease to remove the 5'-end side non-translation region of the EBNA-2 DNA, and the removed site was ligated with an *Eco*RI linker having a length of 8 nucleotides. The thus constructed plasmid pUC-RBΔ1 has an *Eco*RI site just in front of the translation initiation codon of the EBNA-2 DNA.

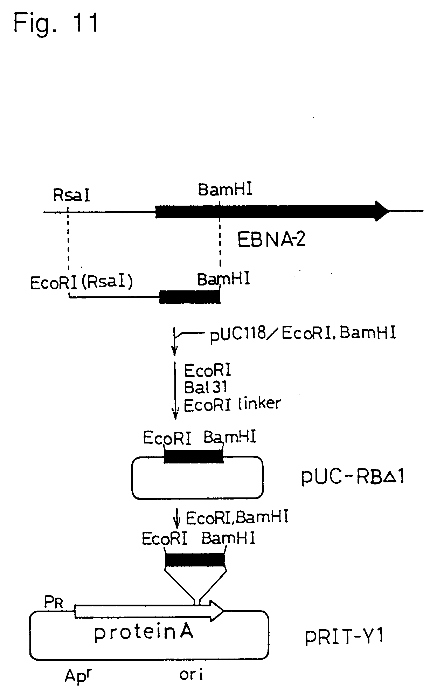

Next, the plasmid pUC-RBΔ1 was digested with *Eco*RI and *Bam*HI and inserted into an *Eco*RI-*Bam*HI site of plasmid pRIT2T (Nilsson, B. *et al.*, already cited in the foregoing), a site which locates on the C-terminal-corresponding side of a sequence that encodes the IgG binding region of protein A. In this manner, a plasmid pRIT-Y1 was constructed (Fig. 11).

(2) Isolation of transformant

An *E. coli* strain N4830 was transformed with the just obtained plasmid pRIT-Y1 in the usual way.

(3) Culturing of transformant and recovery of fusion protein

A transformed *E. coli* strain prepared in Example 3-(2) was cultured at 32°C for about 2 hours in LB medium which has been supplemented with 40 μg/ml of ampicillin.

When the strain grew into a cell density of 1-2 x $10^8$ cells/ml, the culture temperature was increased to 45°C. After 20 minutes of shaking culture at the elevated temperature, the culturing was continued at 39°C for 90 minutes.

The thus obtained culture broth was cooled in an ice bath for 5 minutes and then subjected to centrifugation at 8,000 x g for 5 minutes.

The resulting precipitate was suspended in 15 ml of 10 mM tris-HCl buffer (pH 7.5) containing 200 mM NaCl and 1 mM EDTA (to be referred to as "Buffer C" hereinafter). The thus prepared cell suspension was mixed with 1 ml of lysozyme solution (10 mg/ml), allowed to stand still at 0°C for 10 to 15 minutes and then subjected to two cycles of freeze-thawing step using a dry ice-methanol system. Thereafter, the resulting mixture was subjected to ultrasonic treatment and then to ultracentrifugation at 4°C and at 25,000 x g for 30 minutes. The resulting supernatant was recovered to be used as a crude extract.

(4) Purification of fusion protein

The crude extract obtained in Example 3-(2) was fractionated by salting-out techniques. That is, ammonium sulfate was added to the crude extract to a concentration of 40% saturation, and the mixture was allowed to stand still at 4°C for several hours.

The thus treated extract was subjected to centrifugation at 4°C and at 6,000 x g for 30 minutes. The resulting precipitate was dissolved in 50 to 100 ml of 50 mM phosphate buffer (pH 7.4) containing 110 mM NaCl and 0.05% Tween 20 (to be referred to as "Buffer D" hereinafter), and the resulting solution was dialyzed against Buffer D at 4°C to obtain a sample.

Next, an IgG Sepharose 6FF column (5 ml capacity, Pharmacia) was washed with 3 ml of Buffer D and then with 3 ml of 1 M acetate buffer (pH 2.7). After repeating the washing step twice, the column was equilibrated with Buffer D.

About 50 to 100 ml of the sample obtained above by ammonium sulfate salting-out was applied to the thus equilibrated column at a flow rate of 5-8 ml/hr, followed by washing with about 100 ml of Buffer D at a flow rate of 10-20 ml/hr.

After further washing the column with about 20 ml of 50 mM phosphate buffer (pH 7.4) containing 110 mM NaCl, elution was carried out using about 10 ml of 5 mM ammonium acetate and then using 8 ml of 1 M acetate buffer (pH 2.7). The eluates were pooled into protein peak fractions in the same manner as in Example 2-(4). Thereafter, the pooled fractions were dialyzed against 50 mM Tris buffer (pH 7.4), and a portion of the resulting solution was subjected to salting-out techniques in the same manner as described above in which final concentration of ammonium sulfate was changed within the range of from 30 to 40% saturation depending on each pooled fraction. In this way, a fusion protein sample was obtained.

The inventors of the present invention have isolated an *E. coli* strain transformed with a vector containing a DNA fragment coding for the thus obtained EBNA fusion protein, which has been deposited by the present inventors on December 13, 1990, in Fermentation Research Institute, Agency of Industrial Science and Technology, and has been assigned the designation as FERM P-11907, transferred to International Deposit No. BP-3790 (expresses EBNA-2 (Y1)).

(5) Separation of carrier protein

A dialyzed sample obtained in Example 3-(4) was mixed with 60 μg of lysyl endopeptidase (biochemical use, Wako Pure chemical industries, Ltd.), incubated at 37°C for 30 minutes and then subjected to salting-out using 60% saturation ammonium sulfate in the same manner as in Example 3-(4), followed by centrifugation.

The precipitate thus obtained was dissolved in 5 ml of Buffer D containing 0.01 mM of p-amidinophenylmethanesulfonyl fluoride to use as a sample after carrier protein separation.

(6) Purification of EBNA protein

The sample obtained in Example 3-(5) was applied to the aforementioned IgG Sepharose column and washed with 5 ml of Buffer D. About 10 ml of the eluates were pooled and subjected to salting-out using 60% saturation ammonium sulfate in the same manner as in Example 3-(4).

The resulting fraction was subjected to gel filtration using Sephacryl S-200 column to recover an eluted fraction equivalent to around 15 kDa. The 14 kDa fraction was subjected to salting-out using 60% saturation ammonium sulfate in the same manner as in Example 3-(4), followed by dialysis against 50 mM phosphate buffer (pH 7.4) containing 11 mM NaCl to obtain a final sample (protein Y1 containing a portion of EBNA-2).

(7) Production of protein 0.5K

Protein 0.5K was obtained by repeating the procedures described above almost in the same manner except that a subfragment 0.5K was used instead of the subfragment Y1.

(Example 4) Analysis of antigenicity of fusion protein making use of EBV-positive human sera

Reactivities of the fusion protein samples obtained in Example 2 with anti-EBV antibody positive human sera were analyzed by immunoblotting.

Each of the fusion protein samples was subjected to SDS-polyacrylamide gel electrophoresis (Laemmli, U.K., Nature, 227, pp.680 - 685, 1970), protein in the resulting gel was transferred to a nitrocellulose filter (Towbin, H. $et\ al.$, Proc. Natl. Acad. Sci., U.S.A.,76, pp.4350 - 4354, 1979) and the resulting filter was allowed to react with EBV-positive human sera at $37°C$ for 1 hour. After washing, bands on the filter were detected by a color reaction or autoradiography in which a $^{125}I$ marker was used.

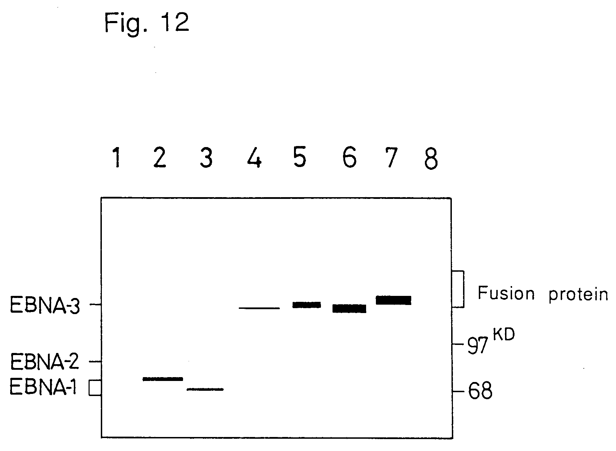

The results are shown in Fig. 12 in which lane 1 is a cell extract of Ramos (an EBV-negative cell line) used as a negative control and lanes 2 and 3 are cell extracts of EBV-positive cell lines Jijoy and Raji, respectively, both used as positive controls. Lane 4 is EBNA-1 fusion protein prepared by introducing subfragment K2, lane 5 is EBNA-2 fusion protein prepared by introducing subfragment H4, lane 6 is EBNA-3 fusion protein prepared by introducing subfragment S8, lane 7 is EBNA-4 fusion protein prepared by introducing subfragment N4 and lane 8 is $\beta$-galactosidase. In Fig. 12, the terms "EBNA-1", "EBNA-2" and "EBNA-3" denoted on the left side of the gel show positions which correspond to reported molecular weights of respective EBNA proteins.

As shown in Fig. 12, it was confirmed that these fusion proteins are possessed of sufficient antigenicities to react with anti-EBV antibody positive human sera.

(Example 5) Preparation and purification of antibodies specific for A1 (EBNA-6)

Antibodies specific for A1 fusion protein were purified from EBV-positive human sera by a small scale affinity chromatography in accordance with the method of Smith, D.E. $et\ al.$ (J. Cell Biol., 99, pp.20 - 28, 1984).

That is, the A1 fusion protein prepared in Example 2-(4) was bound to a nitrocellulose strip and then allowed to react with anti-EBV antibody positive human sera. Thereafter, the antibodies attached to the strip were eluted with glycine-HCl buffer (pH 2.5).

The thus recovered antibodies in the eluate showed specific reaction with a 144 kDa band of EBNA-6 when analyzed by immunoblotting (data not shown). In the immunoblotting analysis, the antibody sample did not react with other EBNA proteins or $\beta$-galactosidase.

The antibody thus obtained can be used in the present invention as a solid phase antibody a and/or a labeled antibody c for use in the measurement of EBNA-6.

(Example 6) Preparation of anti-$\beta$-galactosidase antibody

About 1 mg of $E.\ coli$ $\beta$-galactosidase (Sigma Chemical Co.) was mixed with Freund's complete adjuvant, and the mixture was subcutaneously administered to the dorsal part of rabbits. Thereafter, about 1 mg of the antigen mixed with Freund's incomplete adjuvant was administered three times at intervals of one week. Anti-$\beta$-galactosidase antibodies were recovered from blood samples of the thus treated rabbits by fractionating sera in the blood samples by ammonium sulfate salting-out techniques and then purifying an IgG-containing fraction by DEAE cellulose column chromatography. The antibody thus obtained was digested with immobilized pepsin in accordance with the method of Kino, T.P. $et\ al.$ (Biochemistry, 17, pp.1499 - 1506, 1978) to obtain F(ab')$_2$ which was subsequently dialyzed against PBS$^-$ buffer (pH 7.0) for further use as an antibody solution.

(Example 7) Measurement of anti-EBNA antibody titers making use of EBNA-1 fusion protein

Human serum samples collected from a chronic rheumatoid arthritis (RA) patient and two healthy volunteers were used as test samples, and anti-EBNA-1 antibody titers in the samples were measured making use of EBNA-1 fusion protein containing $\beta$-galactosidase as its carrier protein moiety.

Firstly, the anti-$\beta$-galactosidase antibody (F(ab')$_2$) solution obtained in Example 6 was added to a 96 well microplate (12 ng/well) to carry out coating at 4°C for 24 hours or longer. After washing the resulting plate, 300 $\mu$l of PBS$^-$ containing 2.0% skim milk was added to each well, followed by overnight aftercoating at 4°C. After washing the resulting plate with PBS$^-$, 2 $\mu$g of the K2 (EBNA-1) fusion protein prepared in Example 2 was added to each well and incubated at 37°C for 1 hour. Thereafter, the resulting microplate was washed three times with PBS$^-$ containing 0.05% Tween 20 (to be referred to as "PBST" hereinafter) to obtain a measuring plate.

Each of the serum samples was diluted with PBS$^-$ containing 2.0% skim milk by dilution factors of 2 x 100, $2^3$ x 100, $2^5$ x 100, $2^7$ x 100 and $2^9$ x 100, and the diluted samples were added to the measuring plate (100 $\mu$l/well) and incubated at 37°C for 1 hour. After washing the resulting plate three times with PBST, 100 $\mu$l of 1,000 times diluted solution of horseradish peroxidase-labeled goat anti-human IgG antibody (Fc specific, 240 U/$\mu$l, available from ICN Immuno Biologicals) was added to each well and incubated at 37°C for 1 hour. After washing the resulting plate four times with PBST buffer, enzyme reaction was carried out by adding to the washed plate 100 $\mu$l/well of 0.04% ABTS (2,2'-azino-bis(3-ethylbenzthiazoline sulfate), 0.05% hydrogen peroxide in 2.0% citric acid buffer (pH 4.0), incubating the resulting plate at room temperature for 40 minutes and then adding 100 $\mu$l/well of 0.02% sodium azide solution to stop the reaction.

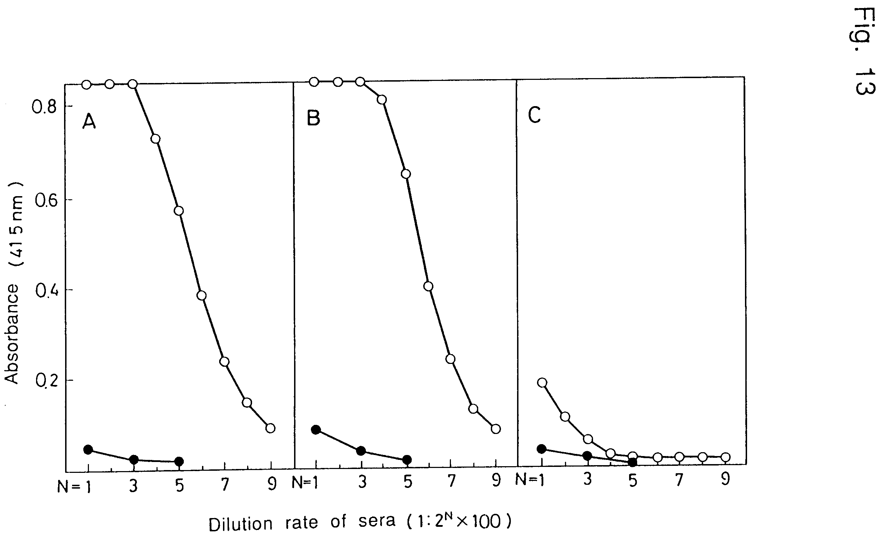

Thereafter, absorbance at 415 nm of the reaction solution in each well was measured in respect to the three serum samples, with the results shown in Fig. 13 (data expressed by O). In the figure, A and C are serum samples of healthy volunteers and B is a sample of RA patient. In Japan, most of adult healthy persons are antibody-positive, because initial infection with EBV occurs generally during infant stage (most of the cases do not show serious symptoms). The results shown in Fig. 13 coincide with such situation. As a control, $\beta$-galactosidase was used instead of EBNA-1 fusion protein (expressed by ● in Fig. 13), but cross-reaction with $\beta$-galactosidase was not found in human sera.

(Example 8) Correlation with ordinary method in terms of anti-EBNA-1 antibody titers

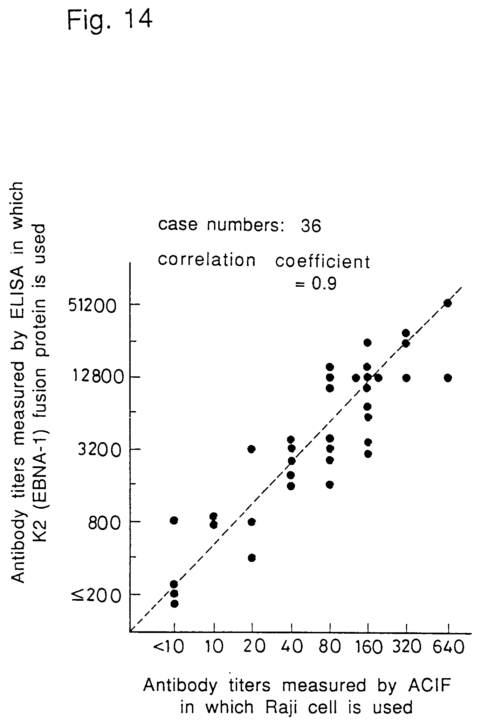

Anti-EBNA-1 antibody titers in 36 serum samples of Japanese adult healthy individuals were measured by the usually used enzyme-linked immunosorbent assay (ELISA) as employed in Example 7 and by an anti-complement immunofluorescence technique (ACIF) in which Raji cells are used. In ELISA, antibody titer was defined as the highest dilution of sera that gave an absorbance of 0.3 at 415 nm after subtracting the absorbance of control antigens. ACIF was carried out in accordance with the method of Reedman and Klein (Int. J. Cancer, 11, pp.499 - 520, 1973). It is known that anti-EBNA-1 antibody titers are mostly measured by the ACIF (Hearing, J.C. et al., Proc. Natl. Acad. Sci., U.S.A., 81, pp.4373 - 4377, 1984). The results are shown in Fig. 14.

As shown in the figure, a correlation coefficient of 0.9 was found between antibody titers measured by ELISA of the present invention and ACIF. Also, based on the results shown in Fig. 14, it was confirmed that the method of the present invention (in the case of an enzyme-labeled system) has a sufficiently broad measuring range.

(Example 9) Measurement of anti-EBNA antibody titers using fusion proteins of EBNA-1 to EBNA-4

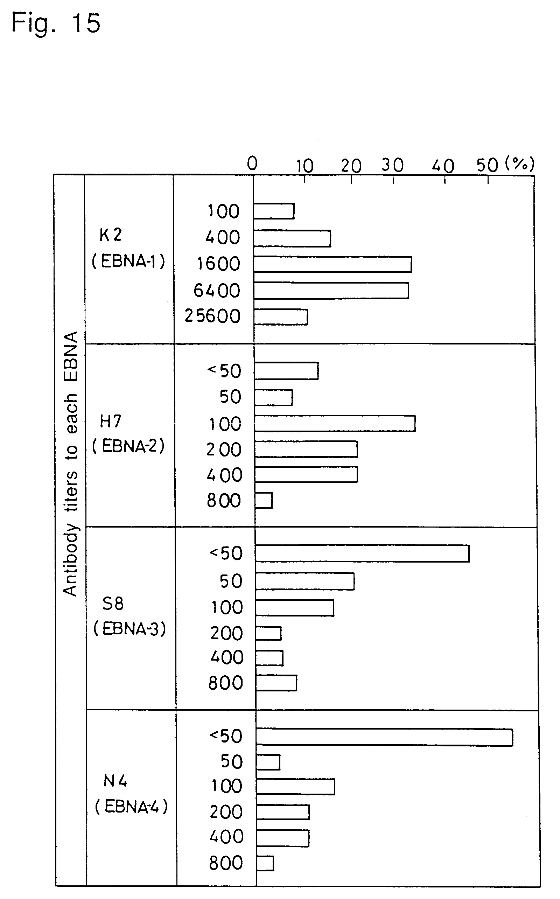

Antibody titers to each EBNA fusion protein in 38 serum samples of Japanese adult healthy individuals were measured in the same manner as in Example 7 except that K2 (EBNA-1) fusion protein, H7 (EBNA-2) fusion protein, S8 (EBNA-3) fusion protein and N4 (EBNA-4) fusion protein all prepared in Example 2 were used. The results are shown in Fig. 15.

As shown in the figure, it was confirmed that levels of antibody titers to these EBNA fusion proteins in sera of adult healthy individuals are different from one another. In this instance, mean IgG antibody titers to EBNA-1, EBNA-2, EBNA-3 and EBNA-4 were found to be 3570, 129, 61 and 60, respectively.

(Example 10) Measurement of anti-EBNA-2 antibody titers using protein Y1 (EBNA-2)

Antibody titers to EBNA-2 in 66 serum samples of Japanese adult healthy individuals were measured using the protein Y1 (EBNA-2) prepared in Example 3-(6).

A 100 $\mu$l portion of a solution of purified protein Y1 (1 $\mu$g/ml PBS) was added to each well of a 96 well microplate to carry out coating at 4°C for 24 hours. Next, 300 $\mu$l of PBS$^-$ containing 2.0% skim milk was added to each well, followed by overnight aftercoating at 4°C. After washing the resulting plate with PBS$^-$, 100 $\mu$l of a serum sample was added to each well and incubated at 37°C for 1 hour. The resulting microplate was washed with PBST and then 100 $\mu$l of 1,000 times diluted solution of horseradish peroxidase-labeled goat anti-human IgG antibody (H-chain and L-chain specific, available from MBL) was added to each well and incubated at 37°C for 1 hour. After washing the resulting plate with PBST, enzyme reaction was carried out by adding the 0.04% ABTS solution described in Example 7 to the washed plate (100 $\mu$l/well), incubating the resulting plate at room temperature for 20 minutes and then adding 100 $\mu$l/well of 0.02% sodium azide solution to stop the reaction. Thereafter, absorbance of the reaction solution in each well was measured at 415 nm. The antibody titer was defined as the highest dilution of sera that gave an absorbance of 0.2 at 415 nm.

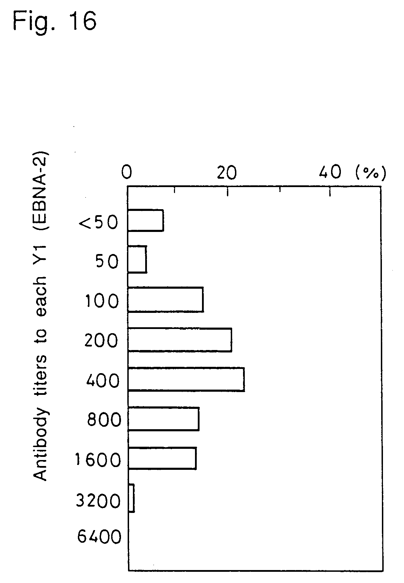

Distribution of IgG antibody titers to the protein Y1 in 66 serum samples of healthy adults is shown in Fig. 16. Mean antibody titer was found to be 268.

(Example 11) Comparison of two anti-EBNA antibody measuring methods in which EBNA-1 fusion protein is used

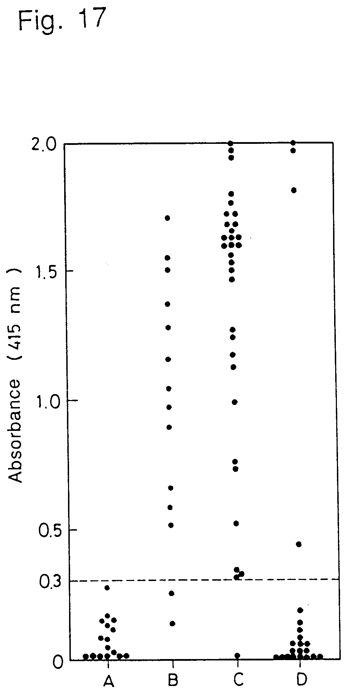

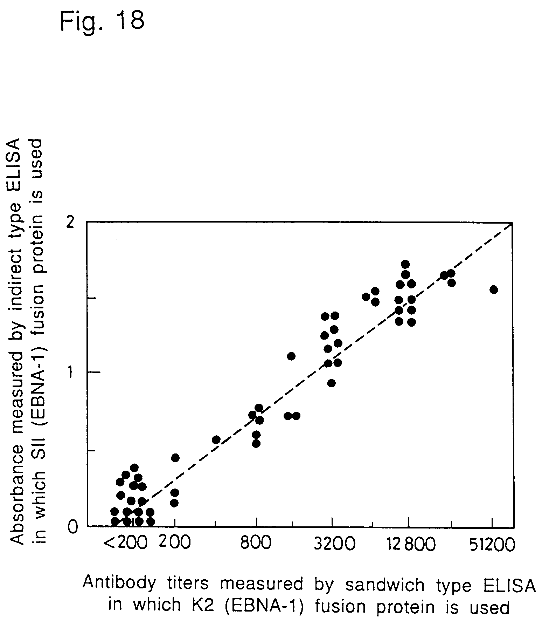

Antibody titers to EBNA-1 in human sera were measured by the sandwich type ELISA described in Example 7 and the indirect type ELISA described in Example 10, using K2 (EBNA-1) fusion protein and SII (EBNA-1) fusion protein which have been prepared in Example 2. The results are shown in Figs. 17 and 18.

Fig. 17 shows distribution of absorbances measured at 415 nm as the results of analysis of 200 times diluted serum samples by the sandwich type ELISA. In the figure, serum samples of anti-EB virus capsid antigen (VCA) antibody-negative 16 healthy individuals are used in lane A, serum samples of anti-VCA antibody-positive 6 healthy infants of 1 to 3 years are used in lane B, serum samples of anti-VCA antibody-positive 38 healthy adults are used in lane C and serum samples of 23 patients of infectious mononucleosis are used in lane D.

Fig. 18 shows a correlation between the two methods in terms of measured values of the same samples.

As is evident from the results shown in Fig. 17, antibody titers to EBNA-1 are low in the case of the anti-VCA antibody-negative samples. Also, as is evident from the results shown in Fig. 18, measured values by the two methods have excellent correlation.

Thus, it is apparent that there have been provided, in accordance with the present invention, methods for the measurement of anti-EBNA antibodies and measuring kits for use in the measurement of anti-EBNA antibodies.

Development of the method and kit of the present invention for the measurement of anti-EBNA antibodies has rendered possible measurement of antibodies specific for EBNA-1 to EBNA-6 separately from one another.

In addition, since recombinant type EBNA-based proteins are used in the method and kit of the present invention, measurement of anti-EBNA antibodies can be carried out without receiving interference by autoantibodies and the like which is a serious problem in the ordinary method, and the EBNA proteins used in the inventive method and kit have stable properties.

Furthermore, the method and kit of the present invention for the measurement of anti-EBNA antibodies are practical in terms of measurable range and measuring accuracy.

Development of the method and kit of the present invention for the measurement of anti-EBNA antibodies has also rendered possible panel type measurement of a plurality of anti-EBNA antibodies in one test sample. In consequence, the method and kit thus provided by the present invention for simple and accurate measurement of anti-EBNA antibodies will contribute greatly to the elucidation of the still ambiguous reaction mechanism of each EBNA protein.

Introduction and popularization of the inventive method will result in the elucidation of the role of each EBNA in EBV-related diseases and the acquirement of new information on the disease-EBNA relationship. Especially, when correlations between each disease and appearance and changing pattern of antibody titers to each EBNA are found, it is highly possible that measurement of anti-EBNA antibodies can be applied to the definite diagnosis, early diagnosis and therapeutic monitoring of EBV-related diseases, as well as a broad range of immunological inspections, analyses and the like.

Although some preferred embodiments have been described, many modifications and variations may be made thereto in the light of the above teachings. It is therefore to be understood that within the scope of

EP 0 508 427 A2

the appended claims, the invention may be practiced otherwise than as specifically described.

**Claims**

1. A method for measuring anti-EBNA antibodies which comprises using a recombinant type EBNA-based protein.

2. The method for measuring anti-EBNA antibodies according to claim 1 wherein said EBNA-based protein is a protein which contains a portion of EBNA.

3. The method for measuring anti-EBNA antibodies according to claim 1 or 2 wherein said EBNA-based protein is an EBNA fusion protein which contains at least a portion of EBNA and a carrier protein.

4. The method for measuring anti-EBNA antibodies according to claim 3 wherein said carrier protein is $\beta$-galactosidase.

5. The method for measuring anti-EBNA antibodies according to any one of the claims 1 to 4 wherein said method comprises the steps of:
   allowing anti-EBNA antibodies in a test sample to react with a recombinant type EBNA-based protein immobilized on a support through an antibody a,
   allowing said anti-EBNA antibodies to react with an antibody b labeled with a labeling compound, and
   measuring signals generated from said labeling compound contained in an immune complex formed by the above steps which comprises the solid phase antibody a, the recombinant type EBNA-based protein, the anti-EBNA antibody and the labeled antibody b in that order.

6. The method for measuring anti-EBNA antibodies according to any one of the claims 1 to 4 wherein said method comprises the steps of:
   allowing a recombinant type EBNA-based protein to react with an antibody a immobilized on a support,
   allowing said EBNA-based protein to react with anti-EBNA antibodies in a test sample,
   allowing said anti-EBNA antibodies to react with an antibody b labeled with a labeling compound, and
   measuring signals generated from said labeling compound contained in an immune complex formed by the above steps which comprises the solid phase antibody a, the recombinant type EBNA-based protein, the anti-EBNA antibody and the labeled antibody b in that order.

7. The method for measuring anti-EBNA antibodies according to any one of the claims 1 to 4 wherein said method comprises the steps of:
   allowing anti-EBNA antibodies in a test sample to react with a recombinant type EBNA-based protein x immobilized on a support through an antibody a,
   allowing said anti-EBNA antibodies to react with a recombinant type EBNA-based protein y labeled with a labeling compound, and
   measuring signals generated from said labeling compound contained in an immune complex formed by the above steps which comprises the solid phase antibody a, the recombinant type EBNA-based protein x, the anti-EBNA antibody and the labeled recombinant type EBNA-based protein y in that order.

8. The method for measuring anti-EBNA antibodies according to any one of the claims 1 to 4 wherein said method comprises the steps of:
   allowing a recombinant type EBNA-based protein x to react with an antibody a immobilized on a support,
   allowing said EBNA-based protein x to react with anti-EBNA antibodies in a test sample,
   allowing said anti-EBNA antibodies to react with a recombinant type EBNA-based protein y labeled with a labeling compound, and
   measuring signals generated from said labeling compound contained in an immune complex formed by the above steps which comprises the solid phase antibody a, the recombinant type EBNA-based protein x, the anti-EBNA antibody and the recombinant type EBNA-based protein y in that order.

27

9. The method for measuring anti-EBNA antibodies according to any one of the claims 1 to 4 wherein said method comprises the steps of:

allowing anti-EBNA antibodies in a test sample to compete with an antibody c labeled with a labeling compound for a recombinant type EBNA-based protein immobilized on a support through an antibody a, and

measuring signals generated from said labeling compound contained in an immune complex formed by the above step which comprises the solid phase antibody a, the recombinant type EBNA-based protein and the labeled antibody c in that order.

10. The method for measuring anti-EBNA antibodies according to any one of the claims 1 to 4 wherein said method comprises the steps of:

allowing a recombinant type EBNA-based protein to react with an antibody a immobilized on a support,

allowing anti-EBNA antibodies in a test sample to compete with an antibody c labeled with a labeling compound for said EBNA-based protein, and

measuring signals generated from said labeling compound contained in an immune complex formed by the above steps which comprises the solid phase antibody a, the recombinant type EBNA-based protein and the labeled antibody c in that order.

11. The method for measuring anti-EBNA antibodies according to any one of the claims 1 to 4 wherein said method comprises the steps of:

allowing anti-EBNA antibodies in a test sample to react with a recombinant type EBNA-based protein immobilized on a support,

allowing said anti-EBNA antibodies to react with an antibody b labeled with a labeling compound, and

measuring signals generated from said labeling compound contained in an immune complex formed by the above steps which comprises the solid phase recombinant type EBNA-based protein, the anti-EBNA antibody and the labeled antibody b in that order.

12. The method for measuring anti-EBNA antibodies according to any one of the claims 1 to 4 wherein said method comprises the steps of:

allowing anti-EBNA antibodies in a test sample to react with a recombinant type EBNA-based protein x immobilized on a support,

allowing said anti-EBNA antibodies to react with a recombinant type EBNA-based protein y labeled with a labeling compound, and

measuring signals generated from said labeling compound contained in an immune complex formed by the above steps which comprises the solid phase recombinant type EBNA-based protein x, the anti-EBNA antibody and the labeled recombinant type EBNA-based protein y in that order.

13. The method for measuring anti-EBNA antibodies according to any one of the claims 1 to 4 wherein said method comprises the steps of:

allowing anti-EBNA antibodies in a test sample to compete with an antibody c labeled with a labeling compound for a recombinant type EBNA-based protein immobilized on a support, and

measuring signals generated from said labeling compound contained in an immune complex formed by the above step which comprises the solid phase recombinant type EBNA-based protein and the labeled antibody c in that order.

14. The method for measuring anti-EBNA antibodies according to any one of the claims 1 to 4 wherein said method comprises the steps of:

allowing anti-EBNA antibodies in a test sample to react with an antibody b immobilized on a support,

allowing said anti-EBNA antibodies to react with a recombinant type EBNA-based protein,

allowing said EBNA-based protein to react with an antibody a labeled with a labeling compound, and

measuring signals generated from said labeling compound contained in an immune complex formed by the above steps which comprises the solid phase antibody b, the anti-EBNA antibody, the recombinant type EBNA-based protein and the labeled antibody a in that order.

**15.** The method for measuring anti-EBNA antibodies according to any one of the claims 1 to 4 wherein said method comprises the steps of:

allowing anti-EBNA antibodies in a test sample to react with an antibody b immobilized on a support,

allowing said anti-EBNA antibodies to react with a recombinant type EBNA-based protein labeled with a labeling compound, and

measuring signals generated from said labeling compound contained in an immune complex formed by the above steps which comprises the solid phase antibody b, the anti-EBNA antibody and the labeled recombinant type EBNA-based protein in that order.

**16.** The method for measuring anti-EBNA antibodies according to any one of the claims 1 to 4 wherein said method comprises the steps of:

allowing anti-EBNA antibodies in a test sample to compete with an antibody c labeled with a labeling compound for a recombinant type EBNA-based protein immobilized on a support through antibodies b and a, and

measuring signals generated from said labeling compound contained in an immune complex formed by the above step which comprises the solid phase antibody b, the antibody a, the recombinant type EBNA-based protein and the labeled antibody c in that order.

**17.** The method for measuring anti-EBNA antibodies according to any one of the claims 1 to 4 wherein said method comprises the steps of:

allowing a recombinant type EBNA-based protein to react with an antibody a immobilized on a support through an antibody b,

allowing anti-EBNA antibodies in a test sample to compete with an antibody c labeled with a labeling compound for said EBNA-based protein, and

measuring signals generated from said labeling compound contained in an immune complex formed by the above steps which comprises the solid phase antibody b, the antibody a, the recombinant type EBNA-based protein and the labeled antibody c in that order.

**18.** The method for measuring anti-EBNA antibodies according to any one of the claims 1 to 4 wherein said method comprises the steps of:

allowing an antibody a to react with an antibody b immobilized on a support,

allowing said antibody a to react with a recombinant type EBNA-based protein,

allowing anti-EBNA antibodies in a test sample to compete with an antibody c labeled with a labeling compound for said EBNA-based protein, and

measuring signals generated from said labeling compound contained in an immune complex formed by the above steps which comprises the solid phase antibody b, the antibody a, the recombinant type EBNA-based protein and the labeled antibody c in that order.

**19.** The method for measuring anti-EBNA antibodies according to any one of the claims 5 to 10, 14, and 16 to 18 wherein said antibody a is an anti-EBNA antibody.

**20.** The method for measuring anti-EBNA antibodies according to any one of the claims 5 to 10, 14 and 16 to 18 wherein said antibody a is an anti-carrier protein antibody.

**21.** The method for measuring anti-EBNA antibodies according to any one of the claims 5, 6, 11 and 14 to 18 wherein said antibody b is an anti-human immunoglobulin antibody.

**22.** The method for measuring anti-EBNA antibodies according to any one of the claims 5 to 21 wherein said labeling compound is an enzyme.

**23.** The method for measuring anti-EBNA antibodies according to any one of the claims 5 to 21 wherein said labeling compound is a fluorescent material.

**24.** The method for measuring anti-EBNA antibodies according to anyone of the claims 5 to 21 wherein said labeling compound is a chemiluminescent material.

**25.** The method for measuring anti-EBNA antibodies according to any one of the claims 5 to 21 wherein said labeling compound is a radioactive isotope.

26. An anti-EBNA antibody measuring kit which contains a recombinant type EBNA-based protein and is used for effecting the measuring method of any one of the claims 1 to 25.

27. The anti-EBNA antibody measuring kit according to claim 26 which is used for effecting the measuring method of claim 5, wherein an antibody a and a recombinant type EBNA-based protein are made into a solid phase on a support through binding of said antibody a to said support and binding of said EBNA-based protein to said antibody a, and an antibody b labeled with a labeling compound is set to said support.

28. The anti-EBNA antibody measuring kit according to claim 26 which is used for effecting the measuring method of claim 6, wherein an antibody a is made into a solid phase on a support, and a recombinant type EBNA-based protein and an antibody b labeled with a labeling compound are set to said support.

29. The anti-EBNA antibody measuring kit according to claim 26 which is used for effecting the measuring method of claim 5, wherein an antibody a and a recombinant type EBNA-based protein are immobilized on a support through binding of said antibody a to said support and binding of said EBNA-based protein to said antibody a, and an antibody b labeled with a labeling compound is separately packaged.

30. The anti-EBNA antibody measuring kit according to claim 26 which is used for effecting the measuring method of claim 6, wherein an antibody a is immobilized on a support, and a recombinant type EBNA-based protein and an antibody b labeled with a labeling compound are separately packaged.

31. The anti-EBNA antibody measuring kit according to claim 26 which is used for effecting the measuring method of claim 7, wherein an antibody a and a recombinant type EBNA-based protein x are immobilized on a support through binding of said antibody a to said support and binding of said EBNA-based protein x to said antibody a, and a recombinant type EBNA-based protein y labeled with a labeling compound is set to said support.

32. The anti-EBNA antibody measuring kit according to claim 26 which is used for effecting the measuring method of claim 8, wherein an antibody a is immobilized on a support, and a recombinant type EBNA-based protein x and a recombinant type EBNA-based protein y labeled with a labeling compound are set to said support.

33. The anti-EBNA antibody measuring kit according to claim 26 which is used for effecting the measuring method of claim 7, wherein an antibody a and a recombinant type EBNA-based protein x are immobilized on a support through binding of said antibody a to said support and binding of said EBNA-based protein x to said antibody a, and a recombinant type EBNA-based protein y labeled with a labeling compound is separately packaged.

34. The anti-EBNA antibody measuring kit according to claim 26 which is used for effecting the measuring method of claim 8, wherein an antibody a is immobilized on a support, and a recombinant type EBNA-based protein x and a recombinant type EBNA-based protein y labeled with a labeling compound are separately packaged.

35. The anti-EBNA antibody measuring kit according to claim 26 which is used for effecting the measuring method of claim 9, wherein an antibody a and a recombinant type EBNA-based protein are immobilized on a support through binding of said antibody a to said support and binding of said EBNA-based protein to said antibody a, and an antibody c labeled with a labeling compound is adsorbed to said support or bound to said EBNA-based protein.

36. The anti-EBNA antibody measuring kit according to claim 26 which is used for effecting the measuring method of claim 10, wherein an antibody a is immobilized on a support, and a recombinant type EBNA-based protein and an antibody c labeled with a labeling compound are set to said support with or without mutual binding.

37. The anti-EBNA antibody measuring kit according to claim 26 which is used for effecting the measuring method of claim 9, wherein an antibody a and a recombinant type EBNA-based protein are immobilized on a support through binding of said antibody a to said support and binding of said EBNA-based protein to said antibody a, and an antibody c labeled with a labeling compound is separately packaged.

38. The anti-EBNA antibody measuring kit according to claim 26 which is used for effecting the measuring method of claim 10, wherein an antibody a is immobilized on a support, and a recombinant type EBNA-based protein and an antibody c labeled with a labeling compound are separately packaged with or without mutual binding.

39. The anti-EBNA antibody measuring kit according to claim 26 which is used for effecting the measuring method of claim 11, wherein a recombinant type EBNA-based protein is immobilized on a support, and an antibody b labeled with a labeling compound is set to said support.

40. The anti-EBNA antibody measuring kit according to claim 26 which is used for effecting the measuring method of claim 11, wherein a recombinant type EBNA-based protein is immobilized on a support, and an antibody b labeled with a labeling compound is separately packaged.

41. The anti-EBNA antibody measuring kit according to claim 26 which is used for effecting the measuring method of claim 12, wherein a recombinant type EBNA-based protein x is made into a solid phase on a support, and a recombinant type EBNA-based protein y labeled with a labeling compound is set to said support.

42. The anti-EBNA antibody measuring kit according to claim 26 which is used for effecting the measuring method of claim 12, wherein a recombinant type EBNA-based protein x is immobilized on a support, and a recombinant type EBNA-based protein y labeled with a labeling compound is separately packaged.

43. The anti-EBNA antibody measuring kit according to claim 26 which is used for effecting the measuring method of claim 13, wherein a recombinant type EBNA-based protein is immobilized on a support, and an antibody c labeled with a labeling compound is set to said support or bound to said EBNA-based protein.

44. The anti-EBNA antibody measuring kit according to claim 26 which is used for effecting the measuring method of claim 13, wherein a recombinant type EBNA-based protein is bound to a support, and an antibody c labeled with a labeling compound is separately packaged.

45. The anti-EBNA antibody measuring kit according to claim 26 which is used for effecting the measuring method of claim 14, wherein an antibody b is immobilized on a support, and a recombinant type EBNA-based protein and an antibody a labeled with a labeling compound are set to said support with or without mutual binding.

46. The anti-EBNA antibody measuring kit according to claim 26 which is used for effecting the measuring method of claim 14, wherein an antibody b is immobilized on a support, and a recombinant type EBNA-based protein and an antibody a labeled with a labeling compound are separately packaged with or without mutual binding.

47. The anti-EBNA antibody measuring kit according to claim 26 which is used for effecting the measuring method of claim 15, wherein an antibody b is immobilized on a support, and a recombinant type EBNA-based protein labeled with a labeling compound is set to said support.

48. The anti-EBNA antibody measuring kit according to claim 26 which is used for effecting the measuring method of claim 15, wherein an antibody b is immobilized on a support, and a recombinant type EBNA-based protein labeled with a labeling compound is separately packaged.

49. The anti-EBNA antibody measuring kit according to claim 26 which is used for effecting the measuring method of claim 16, wherein an antibody b, an antibody a and a recombinant type EBNA-based protein are immobilized on a support through binding of said antibody b to said support, binding of said antibody a to said antibody b and binding of said EBNA-based protein to said antibody a, and an antibody c labeled with a labeling compound is set to said support or bound to said EBNA-based protein.

50. The anti-EBNA antibody measuring kit according to claim 26 which is used for effecting the measuring method of claim 17, wherein an antibody b and an antibody a are immobilized on a support through binding of said antibody b to said support and binding of said antibody a to said antibody b, and a recombinant type EBNA-based protein and an antibody c labeled with a labeling compound are set to said support with or without mutual binding.

51. The anti-EBNA antibody measuring kit according to claim 26 which is used for effecting the measuring method of claim 18, wherein an antibody b is immobilized on a support, and an antibody a, a recombinant type EBNA-based protein and an antibody c labeled with a labeling compound are set to said support with or without mutual binding.

52. The anti-EBNA antibody measuring kit according to claim 26 which is used for effecting the measuring method of claim 16, wherein an antibody b, an antibody a and a recombinant type EBNA-based protein are immobilized on a support through binding of said antibody b to said support, binding of said antibody a to said antibody b and binding of said EBNA-based protein to said antibody a, and an antibody c labeled with a labeling compound is separately packaged.

53. The anti-EBNA antibody measuring kit according to claim 26 which is used for effecting the measuring method of claim 17, wherein an antibody b and an antibody a are immobilized on a support through binding of said antibody b to said support and binding of said antibody a to said antibody b, and a recombinant type EBNA-based protein and an antibody c labeled with a labeling compound are separately packaged with or without mutual binding.

54. The anti-EBNA antibody measuring kit according to claim 26 which is used for effecting the measuring method of claim 18, wherein an antibody b is immobilized on a support, and an antibody a, a recombinant type EBNA-based protein and an antibody c labeled with a labeling compound are separately packaged with or without mutual binding.

55. The panel type anti-EBNA antibody measuring kit according to any one of the claims 26 to 54 wherein at least two types of proteins are used as said EBNA-based protein, which are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein and a recombinant type EBNA-6-based protein.

56. The panel type anti-EBNA antibody measuring kit according to any one of the claims 27 to 29, 35 to 37, 39, 40, 43 to 45, 47 and 49 to 52 wherein at least two types of proteins are used as said EBNA-based protein, which are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein and a recombinant type EBNA-6-based protein, and the selected EBNA-based proteins are arranged on said support separately from one another.

57. The panel type anti-EBNA antibody measuring kit according to any one of the claims 31, 32 and 41 wherein at least two types of proteins are used as said EBNA-based proteins x and y, which are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein and a recombinant type EBNA-6-based protein, each type of the selected EBNA-based proteins is used as a combination of EBNA-based proteins x and y, and the combination type EBNA-based proteins are arranged on said support separately from one another.

58. The panel type anti-EBNA antibody measuring kit according to claim 33 or 42 wherein at least two types of proteins are used as said EBNA-based proteins x and y, which are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein and a recombinant type EBNA-6-based protein, each type of the selected EBNA-based proteins is used as a combination of EBNA-based proteins x and y, and the EBNA-based protein x counterparts of the combination type EBNA-based proteins are arranged on said support separately from one another and the EBNA-based protein y counterparts of the combination type EBNA-based proteins are individually packaged.

59. A panel type method for measuring anti-EBNA antibodies which comprises measuring at least two different types of anti-EBNA antibody in one test sample simultaneously and individually by using at least two different types of EBNA-based protein.

60. The panel type method according to claim 59 wherein said at least two different types of EBNA-based protein are selected from the group consisting of a recombinant type EBNA-1-based protein, a recombinant type EBNA-2-based protein, a recombinant type EBNA-3-based protein, a recombinant type EBNA-4-based protein, a recombinant type EBNA-5-based protein, and a recombinant type EBNA-6-based protein.

# Fig. 1

Fig. 2

(A)          (B)

Fig. 3

(A)          (B)

Fig. 4

(A)          (B)

Fig. 5

(A)          (B)

Fig. 6

(A)

(B)

Fig. 7

(A)

(B)

Fig. 8

(A)

(B)

Fig. 9

(A)

(B)

Fig. 10

(A)    (B)

## Fig. 11

Fig. 12

Fig. 13

Fig. 14

# Fig. 15

Fig. 16

Fig. 17

Fig. 18

Antibody titers measured by sandwich type ELISA
in which K2 (EBNA-1) fusion protein is used